# EUROPEAN PATENT APPLICATION

(11) **EP 2 604 265 A1**
(43) Date of publication of application: **19.06.2013**
(21) Application number: 11382386.8
(22) Date of filing: 17.12.2011
(51) Int. Cl.: A61K 31/41, A61P 25/06

(54) **P2x7 antagonists as frontline or adjunctive treatment against status epilepticus**

(71) Applicant: Royal College of Surgeons in Ireland (RCSI), Dublin 2 (IE); Universidad Complutense De Madrid, 28040 Madrid (ES)
(72) Inventor: Henshall, David C., Dublin 2 (IE); Engel, Tobias, Dublin 2 (IE); Díaz Hernández, Miguel, 28040 Madrid (ES); Miras Portugal, María Terasa, 28040 Madrid (ES)
(74) Representative: Elzaburu Marquez, Alberto

(57) **Abstract**

P2X7 antagonists as frontline or adjunctive treatment against status epilepticus. It is shown that P2X7 antagonists can be used for frontline or adjunctive treatment for the ameloration or termination of status epilepticus and neonatal seizures, especially refractory status epilepticus or neonatal seizures. Combination with current anticonvulsants, particularly GABA-based anticonvulsants such as benzodiazepines or barbiturates, results in the effective termination of status epilepticus, even when it is refractory.

## Description

### TECHNICAL FIELD

The invention belongs to the technical field of the new uses of compounds for treatment of disorders with no known therapy or whose current therapy has undesired effects that should be avoided. Particularly, the invention refers to P2X7 antagonists for frontline or adjunctive treatment of status epilepticus and neonatal seizures, especially refractory status epilepticus or neonatal seizures.

### BACKGROUND OF THE INVENTION

*Status epilepticus* (SE) is a major neurological emergency that is associated with high mortality and morbidity, including permanent damage to the brain. It is not a unitary condition, but rather a collection of disorders that share persistent or recurring seizures. The seizures may be a manifestation of epilepsy, new or persistent, or may occur in the course of another neurological or medical illness, never to return.

Most epileptic seizures last no more than a minute. Occasionally, however, a seizure does not spontaneously terminate, and instead continues. When seizures fail to self-terminate they can develop into *status epilepticus. Status epilepticus* is defined as a single seizure lasting 30 min or more, or as two or more seizures without complete recovery, although shorter operational definitions are now used (Rona et al., 2005; Wasterlain and Chen, 2006), so that single seizures lasting more than 5 min without recovery can be considered *status epilepticus.* It has an incidence of at least 20/100,000 population. *Status epilepticus* is the most common neurological emergency in children, with an incidence of 17 - 23 per 100,000 population (Chin et al., 2006), the immature brain being more susceptible to seizure development (Haut et al., 2004) as well as more prone to immediate and long-term adverse neurological consequences.

*Status epilepticus* occurs when the normal mechanisms in the brain for seizure termination fail. These changes include decreased γ-aminobutyric acid (GABA)_{A} receptor density (Naylor et al., 2005) and increased glutamate receptor density (Wasterlain and Chen, 2008; Wasterlain et al., 2009). Changes to sodium channels, pH-sensitive ion channels, reductions in extracellular adenosine and altered glial function, and occurs when the normal mechanisms in the brain for seizure termination fail. These changes include decreased γ-aminobutyric acid GABA_{A} receptor density (Naylor et al., 2005) and increased glutamate receptor density (Wasterlain and Chen, 2008; Wasterlain et al., 2009). Changes to sodium channels, pH-sensitive ion channels, reductions in extracellular adenosine and altered glial function, also contribute.

SE is a neurological emergency that can be profoundly damaging to the brain, producing hippocampal and extra-hippocampal neuron loss in humans (DeGiorgio et al., 1992; Fujikawa et al., 2000)

Another neurological disorder that seems to be associated to GABA_{A} receptors, similarly to SE, is that of seizures in neonates. Neonatal seizures - seizures during the period from birth until the end of the first month - are more common than at any other time of life; up to 3.5 per 1000 births. Seizures in neonates are a very serious problem and are linked to poor neurological outcomes including cerebral palsy, lowered IQ and later-life epilepsy. Neonatal seizures pose a particular challenge to diagnose and are frequently missed. Their cause seems to be closely related to that of adult *status epilepticus,* since they seem to be associated to the presence of a benzodiazepine-insensitive GABA_{A} subunit during brain development, also linked to the depolarizing effects of GABA in the immature brain. Indeed, seizures in neonates are often recurrent and/or prolonged, being considered then as neonatal *status epilepticus,* which is associated with poor outcome in the neonatal population. There is no consensus, however, in the definition of neonatal *status epilepticus,* or even, if the concept can be applied to neonates or only neonatal seizures exist: while some authors (Pisani et al., 2007) apply for neonatal *status epilepticus* the conventional definition (a single seizure lasting 30 min or more, or two or more seizures without complete recovery), other authors (Lawrence and Inder, 2010) consider that electroencephalographic criteria are more appropriate, citing as better definition any electrographic recording with seizure activity > 50% in the length of the recording time.

Critically, no drug has been developed specifically for neonatal seizures and there has been little progress in their effective treatment. Indeed, phenobarbitone, the most commonly used drug for treating neonatal seizures, fails to stop neonatal seizures in over 50 % of cases. Lorazepam, the first-line therapy for *status epilepticus* in children and adults, also commonly fails to stop neonatal seizures. Thus, there is a need to identify novel approaches to treating neonatal seizures, particularly when they are recurrent and/or prolonged.

Therapy for SE, in turn, should proceed simultaneously along three fronts: termination of SE, prevention of recurrence, and treatment of complications. Management of SE can be challenging. Clinical data indicate that spontaneous cessation of generalized convulsive seizures is unlikely after 5 min and, therefore, acute treatment is required; in the case of children spontaneously remitting seizures may last slightly longer, even up to 12 minutes, but after this duration treatment will be required to terminate the seizure.

Frontline pharmacotherapy for SE is with anticonvulsants such as benzodiazepines, with lorazepam being the drug of first choice (Lowenstein, 2005; Chen and Wasterlain, 2006): 0.1 mg/kg lorazepam, given intravenously over 30-60 seconds, is the most common treatment. Lorazepam is usually the benzodiazepine of choice for initial treatment due to its long (2-8 hour) duration of action and rapid onset of effect, thought to be due to its high affinity for receptors of the brain's main inhibitory neurotransmitter, GABA, whose effect is enhanced by lorazepam. If lorazepam is not available, or intravenous access is not possible, then diazepam should be given: 0.15 mg/kg diazepam is also a common treatment. Barbiturates (also compounds whose action is thought to be due to their affinity for GABA_{A} receptors) and a selection of anti-epileptic drugs including phenytoin or other hydantoin derivatives are also used (Lowenstein, 2005; Wasterlain and Chen, 2008), particularly when the seizure continues after ten minutes; then, a common treatment is repeating the same dose of lorazepam/diazepam repeated and administering 18 mg/kg phenytoin over 20 minutes. Cardiac monitoring is a must when phenytoin is administered intravenously. If the seizure continues after ten minutes, further non-anesthetizing anesthetic can be considered, e.g. phenobarbital 20 mg/kg given at 50 mg/min, and a further dose of lorazepam, 4-8 mg stat dose; the patient is usually transferred to intensive care setting in this condition. When conventional anticonvulsant treatment fails, a rapid sequence induction of anesthesia using, for instance, thiopentone 4 mg/kg iv, as a bolus over 20 seconds, is administered, then further boluses of 50 mg until seizures are controlled, continuing infusion which produces a burst suppression pattern on electroencephalogram (EEG).

Many patients respond to the first or second dose of anticonvulsants. But high or repeated doses of anticonvulsants are often required to ensure seizure cessation, with associated risks to life from respiratory depression and coma (Lowenstein, 2005; Wasterlain and Chen, 2008). Barbiturates are used in many cases to induce barbituric comma, particularly when the seizures have to be stopped immediately: it must be remembered that attention to the fundamentals of airway, breathing and circulation must not be neglected and, frequently, the most efficient way to control the airway and respiration is rapid pharmacological termination of SE. The more time a patient spends in continuous seizures, the harder it becomes to terminate the seizures with current drugs, particularly anticonvulsants. Thus, if SE interruption is not prompt, the responsiveness to anticonvulsants declines, and seizures become pharmacorefractory in 10-40 % of SE patients (Lowenstein, 2005). It is common to refer to that situation as refractory status epilepticus (RSE). The scientific basis underlying the drop in efficacy of anticonvulsants (particularly benzodiazepines and barbiturates) is not fully understood, but it appears to be due to desensitization or downregulation of the GABA receptor in the brain (Lowenstein, 2005; Wasterlain et al., 2009).

For some practitioners, the failure of lorazepam alone is considered to be enough to classify a case of SE as refractory; for others, failure of any one of the standard regimes of SE treatment (lorazepam, phenobarbital, diazepam followed by phenytoin, phenytoin alone) should constitute refractory status epilepticus. Other authors require failure of two or three conventional anticonvulsants before deeming SE refractory. Nevertheless, it can be considered that none of the conventional anticonvulsants has a useful role in terminating RSE. The most important therapies are general anesthetic agents, or other drugs used at higher than normal doses to exploit their general anesthetic effects. However, concerns about the adverse hemodynamic and immunological effect of anesthetic doses of effective compounds such as pentobarbital and thiopental has led many investigators to search for less toxic agents.

Some researchers have tried to enhace the efficacy of benzodiazepines in the treatment of status epilepticus, in order to counteract the decrease of the sensibility to benzodiazepines associated to the increase in the duration of the seizures. Glucocorticoid receptor antagonists, such as mifepristone, have been proposed for that object in International Application WO2006/060736. The asssays carried out with rat models set out in said International Application confirm that treatment of rats with mifepristone increases efficiency of diazepam, both when mifepristone is administered before the onset of SE or when the administration takes place once the onset of SE has occurred. Analogously, other compounds that could also potentiate the effect of benzodiazepines are being sought.

Glutamate receptor antagonists appear to remain effective anticonvulsants during SE (Wasterlein and Chen, 2008), but concerns over safety (Ubogo et al., 2003) necessitate identification of further targets.

New anticonvulsant drugs may also have an important role in RSE treatment. As GABAergic failure seems to be associated with the loss of efficacy of tradional anticonvulsants in the treatment of both RSE and neonatal seizures, it should be advisable to search for drugs useful for neonatal seizures and/or RSE management whose targets are not GABA receptors, and finding drugs with anticonvulsant properties that work through a completely different mechanism. Moreover, as previously mentioned, termination of seizures should not be the only goal to achieve, but prevention of recurrence should also be another important objective to accomplish.

Adenosine 5'-triphosphate (ATP) is an important neurotransmitter and gliotransmitter in the nervous system (Burnstock, 2007). ATP is stored and released in response to neuronal and glial activation and its myriad effects include modulation of synaptic strength, glia-neuron communication and cell death (Burnstock, 2007). Under normal conditions, extracellular ATP is rapidly removed from the synaptic cleft by the action of ectonucleotidases. While intracellular ATP levels are depleted during SE, extracellular ATP concentrations actually increase during sustained electrical activity, following tissue trauma and under inflammatory conditions. ATP acts as a fast neurotransmitter via ligand-gated ionotropic P2X receptors (Edwards et al., 1992; Evans et al., 1992). P2X receptors are present in the brain, including at excitatory synapses, and have been implicated in neurodegenerative and neuroimmune disorders (Franke et al., 2006; Burnstock, 2008). Particular interest has emerged in the P2X7 receptor (P2X7R) subtype. P2X7R, which can gate calcium as well as sodium, has been proposed to enhance glutamate release, mediate death of neurons and to have trophic effects on microglia. This receptor might play a role in the development and progression of certain clinical disorders such as chronic inflammation, neurodegeneration or chronic pain. Accordingly, their antagonists have been suggested for the treatment of said disorders.

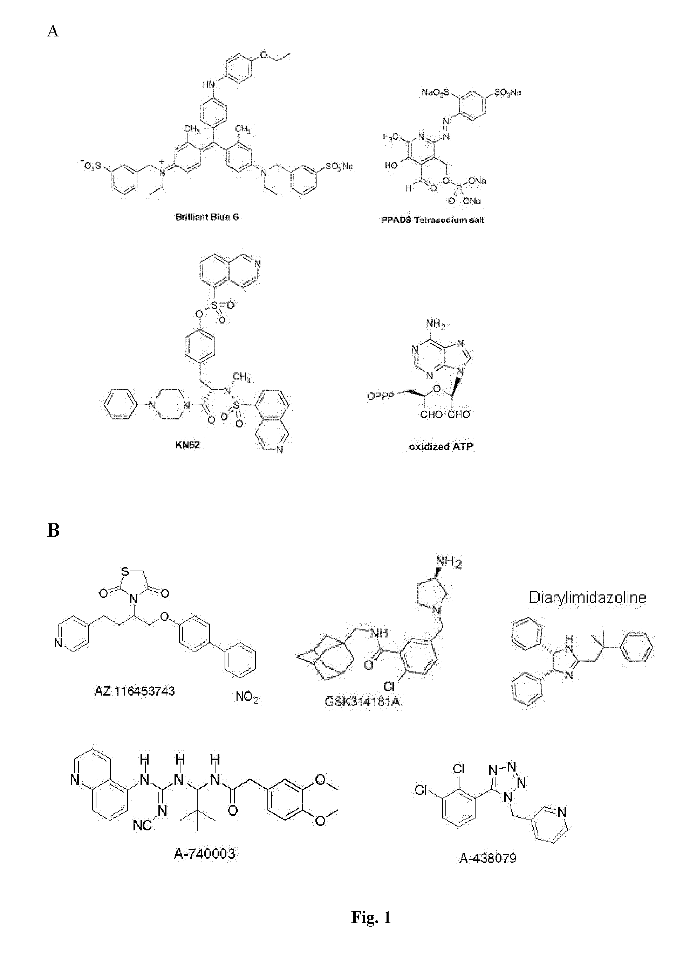

According to North (North RA, 2002), available P2X7R antagonists can be classified into four main groups. The first group contains ions, such as calcium, copper, magnesium, zinc, and protons, all of which inhibit ATP-evoked currents through the P2X7 receptor channel. The second group includes generic or non-selective P2X receptor antagonists such as oxidized ATP (o-ATP: ATP 2',3'-dialdehyde), pyridoxal-phosphate-6-azophenyl-2',4'-disulfonate (PPADS), and Brilliant Blue G (BBG). Third, are the compounds that contain two large cationic cations, i.e. the isoquinoline KN-62 (1-*N,O*-bis[5-isoquinolinesulfonyl]-*N-*methyl-L-tyrosyl)-4-phenylpiperazine and the imidazole calmidazolium, which function to block currents through P2X7 receptors and thereby prevent their activity. These compounds (KN-62, PPADS (which is considered to be a wide spectrum P2 antagonist), oxidized ATP or Brilliant Blue G) (see panel A of Fig. 1 for their structural formula) can be considered the historically known P2X7 antagonists, BBG being the most specific one, although it also inhibits P2X2 and some P2Y receptors in the low micromolar range. More recently, decavanadate, a polymeric form of vanadate (V₁₀O₂₈)⁶⁻, has been reported to be a reversible and competitive P2X7 receptor antagonist *(Michel et al.,* 2006), although not selective.

Other compounds have been developed in the last years in order to have more specific antagonists: cyclic imides like the compound known as AZ116453743 (3-(1-(3'-nitrobiphenyl-4-yloxy)-4-(pyridine-4-yl)butan-2-yl)thiazolidine-2,4-dione), adamantane amides like the compound known as GSK314181A, aryl carbohydrazides as diarylimidazolines, cyanoguanidines like the compound known as A-740003 ((N-(1-{[cyanoimino)(5-quinolinylamino)methyl]amino}-2,2-dimethylpropyl)-2-(3,4-dimethoxyphenyl)acetamide) or disubstituted tetrazoles like the compound known as A-438079 (3-((5-(2,3-dichlorophenyl)-1*H*-tetrazol-1-yl)methylpyridine) (all of them depicted in Fig. 1) have been the subject of recent publications and revisions (see, for instance, Romagnoli et al., Gunosewoyo et al., Donnelly-Roberts et al.). The compounds belonging to the series of disubstituted tetrazoles and cyanoguanidines seem to be potent and selective P2X7 antagonists.

The group of the disubstituted tetrazoles in particular has been the object of several studies about their activity and other properties such as their solubility.

International Patent Application WO2006086229 discloses the use of compounds of the general Formula I 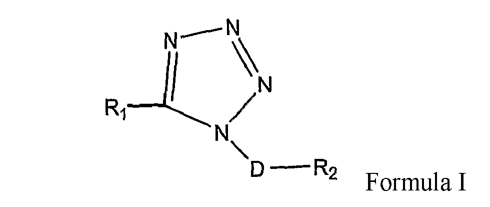 for the treatment of neuropathic pain, chronic inflammatory pain, inflammation, neurodegeneration and for promoting neurodegeneration.

Nelson et al. (Nelson et al., 2006) showed how different variations on a particular case of Formula I, that is, the compound of Formula II, 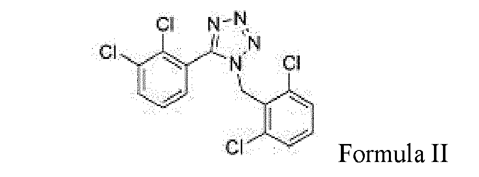 such as the modification of the benzylic groups over the general formula III 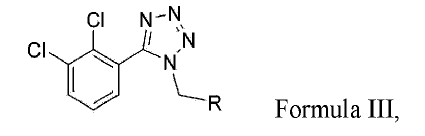 the modification of the phenyl groups in the following formula IV 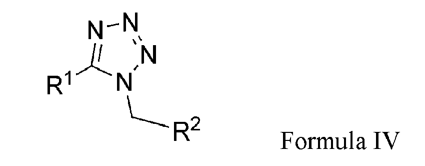 or the modifications of the radical in antagonists which a basic formula with reverse connectivity such as Formula V 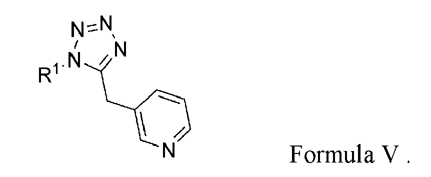 can affect their activity as P2X7R antagonists and some other properties of the obtained compounds.

All modifications seem to give rise to compounds with activity as P2X7R antagonists, although differences in the activity and in some characteristics such as the solubility could be observed.

The use of P2X7R antagonists has been proposed for the treatment of chronic inflammation and pain (Carroll et al., 2009). Most compounds known to inhibit at least some P2X7-mediated central nervous system inflammatory responses fall within the groups of non-selective P2 receptor antagonists and the class of the imidazole derivatives. The use of tetrazole derivatives, such as A-438079 (3-[[5-(2,3-dichlorophenyl)-1*H*-tetrazole-1-yl]methyl]pyridine hydrochloride), for the treatment of neuropathic pain, inflammation and several neurodegenerative disorders, has been the subject of several patent applications, such as WO 2005/111003 and WO2006/086229. In both of them, P2X7 antagonists are presented as compounds useful in methods for the prevention or treatment of neurodegenerative diseases such as Alzheimer's disease, Parkinson's disease, amyotrophic lateral sclerosis, Huntington's disease, dementia with Lewy bodies, multiple sclerosis or diminished CNS function resulting from traumatic brain injury. However, as it is acknowledged in WO 2006/086229, the claimed therapeutic methods are based on the fact that the compounds described in the application are antagonists of the P2X7 receptor, taken together with previous reports on a link between P2X7 receptor and chronic, inflammatory and neuropathic pain (Hatcher et al., 2003), as well as with the results demonstrating a decrease of histological injury and improved recovery of motor function in spinal cord injury animal models treated with antagonists to the P2X7 receptor. None experimental prove is given in WO 2006/086229 of the positive effect of such compounds for neuroregeneration or treatment of neurodegeneration, the *in vitro* secretion of IL-1β and the *in vivo* antinociceptive effect being the only experimental results included in the application. Apart from that, the remaining Examples contain only the description of the synthesis of the specific P2X7 compounds of the application and the assays to verify their structures. This situation is more remarkable in the case of WO2005/111003 where *the in vivo* assays carried out to assess mechanical allodynia and hyperalgesia are placed before Example 1, not mentioning explicitly the compounds used in the assays.

Also the derivatives of tetrahydro trizolo pirazine have been proposed as possible P2X7R antagonists. International application WO2010/125102 describes the synthesis of many of such derivatives, as well as assays to evaluate their potential to antagonize the effects of ATP on P2X7 receptor. An utility of said compounds for the treatment or prevention of neuropathic pain, inflammatory pain, rheumatic arthritis, osteoarthritis, degenerative disorders or epilepsy is also suggested in the application; but this utility is only based on the capability to antagonize ATP effects, assessed through assays in cultures of cells expressing the receptor, and *no in vivo* Example gives any proof to support that possible use of the compounds.

The use of P2X7R antagonists has also been proposed for the treatment of pathologies that associated to the loss of axonal endings and/or absence or reduction of axonal growth. Patent application WO2010/018280 discloses that inhibition of P2X7R induces axon elongation and ramification in the hippocampus, which effect might have a positive influence in the treatment of cognitive pathologies such as Alzheimer's disease, cerebral ischemia, epilepsy, schizophrenia, amyotrophic lateral sclerosis, myasthenia gravis or even the loss of enervation due to an intoxication with botulinum toxin. The Examples contained in said application performed with animal models also demonstrate that P2X7R antagonists able to crossing the blood brain barrier (such as BBG, KN-62 or the inhibitor known as decavanadate, [V₁₀O₂₈]⁶⁻) can be administered intravenously or intraperitoneally to observe an effect in axonal regeneration and/or ramification in the hippocampus, not being necessary their direct delivery to the central nervous system.

P2X7R expression has been reported to be altered in the brain following seizures (Vianna et al., 2002; Rappold et al., 2006; Dona et al., 2009). However, no study has evaluated a possible contribution of P2X7R to seizure generation or progression to a pharmacorefractory state in SE. No *in vivo* study has been carried out either to demonstrate a possible involvement of P2X7R in seizures and, particularly, any possible relation with the pharmacorefractory state.

In this context, the implication of P2X7R in seizure generation or progression and the potential utility of their antagonists to treat or prevent *status epilepticus* and/or neonatal seizures is doubtful. Meanwhile, the need of compounds capable to facilitate, alone or in combination with other drugs, a prompt termination of seizure, and/or capable to prevent their recurrence or the possible damages caused by the seizures, remains to be there. Elucidation of novel molecular mechanisms underlying the pathophysiology of SE or neonatal seizures (particularly, prolonged neonatal seizures or neonatal SE) is critical for it.

The present invention provides a solution to this problem, providing an insight into the molecular mechanisms underlying SE.

### SUMMARY OF THE INVENTION

The present invention refers to the use of P2X7 receptors antagonists for the treatment of status epilepticus in adults, children, infants and neonates, both as a stand-alone medication for ameliorating or terminating *status epilepticus* or as adjunctive treatment with current anticonvulsants, particularly GABA-based anti-convulsants, such as benzodiazepines or barbiturates.

It is based on the finding that pharmacological blockade of P2X7R in an adult mouse and neonatal rat model can significantly shorten and, in some cases, fully terminate *status epilepticus,* both in pre- and post-treatment dosing regimen during SE, and also protected the hippocampus from seizure-induced neuronal death. In mice given a P2X7 antagonist the majority of animals never progressed to the point of continuous seizures where the treatment with lorazepam becomes difficult or impossible; in some cases, the animals became seizure free. The assays set forth below in the Examples of the present application also provide evidences that these animals displayed significantly less brain damage than the vehicle-treated animals which underwent *status epilepticus.* Moreover, in the mouse model, co-injection of a P2X7R antagonist with lorazepam after triggering SE, when electrographic seizure-responsiveness to lorazepam had diminished, implying pharmacoresistance, effectively terminated SE. Thus, P2X7R antagonists are not only a useful compound to terminate or ameliorate the *status epilepticus,* but also a useful tool to treat refractory *status epilepticus* when it is administered with a GABA-based anti-convulsant, especially a benzodiazepine such as lorazepam. Moreover, the assays carried out with the rat model, wherein a P2X7R antagonist capable to cross the blood brain barrier was administered intraperitoneally, support the applicability of P2X7R antagonists capable to cross the blood brain barrier even when they are not administered directly to the central nervous system, which allows to avoid administration routes not very appropriate and accessible in human beings such as the intracerebroventricular route.

Thus, in a first aspect, the invention relates to an antagonist of P2X7 receptor, or a pharmaceutically acceptable salt thereof, for the treatment of *status epilepticus.* The subject in need of the treatment, that is, the subject suffering from *status epilepticus,* can be an adult, a child, an infant, or a neonate. The antagonist can be used alone or in adjunction with an anti-convulsant, particularly a benzodiazepine. The *status epilepticus* can be refractory *status epilepticus,* in which case it is preferred the adjunctive administration of a benzodiazepine. It is preferred the use of those antagonists of P2X7 receptor capable to cross the blood brain barrier, such as A-438079, Brilliant Blue G, KN62 or decavanadate, as well as the pharmaceutically acceptable salts thereof. Lorazepam is the most preferred benzodiazepine.

In a second aspect, the invention refers to a method of use of an antagonist for treating the seizure in the *status epilepticus* in an animal, which method comprises administering to said animal an effective amount of the antagonist of P2X7 receptor or a pharmaceutically acceptable salt thereof. The antagonist can be administered to the animal prior to, contemporaneous with, or subsequent to start of the seizure. The antagonist can be administered to the animal alone or in adjunction with a benzodiazepine. In this last case, the antagonist can be administered prior to, contemporaneous with, or subsequent to the administration of a benzodiazepine. The status epilepticus can be in the pharmacorefractory state (refractory status epilepticus); in this case, the administration in adjunction with a benzodiazepine is preferred. The administration of the antagonist of the P2X7 receptor can be directly to the central nervous system or, preferably, intraperitoneally or intravenously; in the case of intravenous or intraperitoneal administration, antagonists of P2X7 capable to cross the blood brain barrier must be used.

In a third aspect, the invention also refers to a method of use of an antagonist of P2X7R for treating or preventing neonatal seizures or seizures in infants or in children up to five years of age, wherein the antagonist of P2X7R is administered alone or in combination with another approved treatment for neonatal seizures such as a barbiturate, a benzodiazepine, any other approved anti-epileptic drug, or in-trial medication such as bumetanide.

### BRIEF DESCRIPTION OF THE FIGURES

### Fig, 1: Formulae of different antagonists of P2X7 receptor.

**(A):** Formulae of P2X7R traditional antagonists: Brilliant Blue G, PPADS tetrasodium salt, KN62 and oxidized ATP. **(B):** Examples of more recently discovered P2X7R antagonists are depicted: AZ116453743, GSK314181A, diarylimidazoline, A-740003, A-438079.

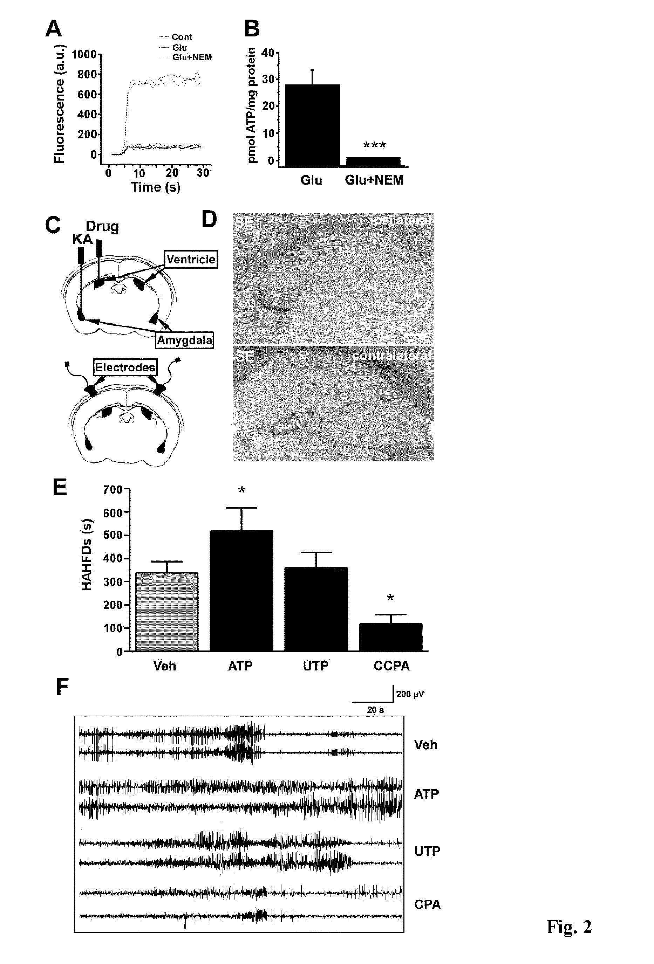

### Fig. 2: Mouse model of SE and effects of ATP:

**(A)** Traces representing extracellular ATP release detected in cultured hippocampal neurons after stimulation with 100 µM glutamate. **(B)** Histogram showing quantification of ATP levels (pmol per mg of protein) in presence or absence of the vesicle fusion inhibitor NEM (N-Ethylmaleimide) (0.5 mM). *(n* = 3 per group). ****p <* 0.0001 **(C)** Schematic showing (top) injection sites for KA (kainic acid) into the amygdala and drug into the ipsilateral ventricle, and (below) cortical electrode placement for EEG. **(D)** Representative photomicrographs showing FJB (Fluoro-Jade B) staining in ipsi- and contra-lateral hippocampus 24h after KA injection (image invert reveals damaged cells as black-stained cells). Note, positive (damaged) cells are in the CA3 region of the ipsi-lateral hippocampus (pointed by arrows). H, hilus; CA1-3, cornu ammonis; CA3a-c, subfields of CA3. **(E)** Average durations of HAHFDs (high amplitude high frequency discharges) during SE: ATP increased seizure time whereas the P2Y-selective agonist UTP had no effect; the adenosine analogue CPA decreased seizure time. **p <* 0.05 versus vehicle (*n* = 8-13 per group). **(F)** Representative EEG traces during SE for each treatment group. Scale bar in D is 250 µm.

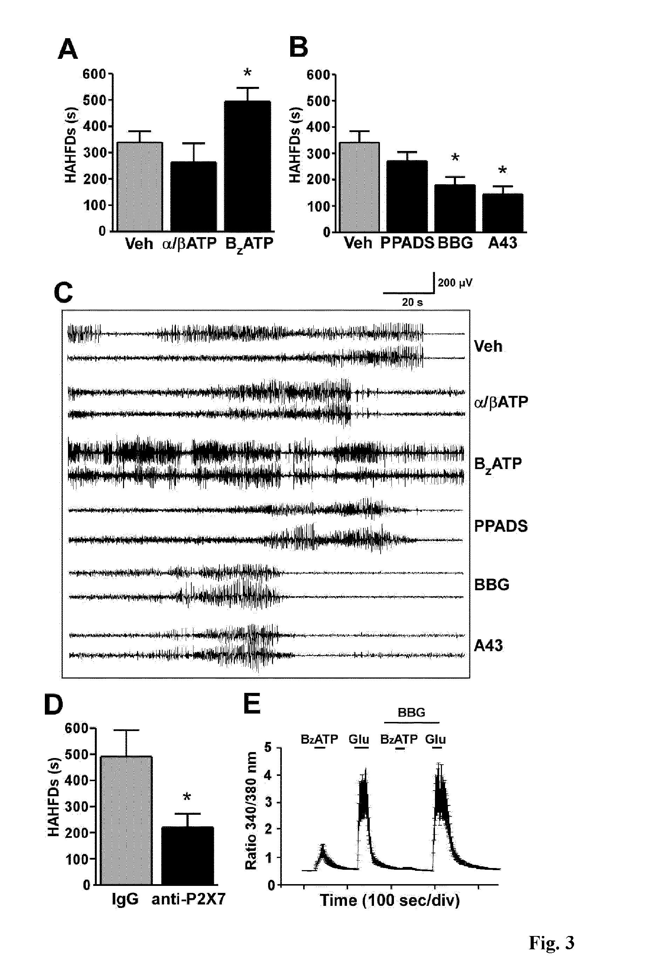

### Fig. 3: P2X7R signalling contributes to seizures during SE

(A) Duration of HAHFDs during SE. Injection of the P2X7R agonist BzATP (benzoylATP) increased seizure time, whereas the P2X wide-spectrum agonist α/β-meATP (α/β-methylATP) had no additive effect *(n* = 8-13 per group). **p <* 0.05 compared to the vehicle, abbreviated "Veh" in the Figure. **(B)** Duration of HAHFDs during SE and effects of P2XR antagonists. Pre-treatment of mice with BBG and A-438079 (A43), but not PPADS, reduced seizures during SE (*n* = 6-14 per group). **(C)** Representative EEG traces from animals during SE and pre-treated with different antagonists and agonists. **(D)** Duration of HAHFDs during SE in mice pre-treated with an antibody against an extracellular epitope of P2X7R. Note, significant decrease of seizure time in mice pre-treated with specific antibody against the P2X7R when compared to animals injected with IgG. *(n* = 6-7 per group; **p <* 0.05). **(E)** Representative traces of evoked intracellular calcium entry, measured by ratiometric dye, in hippocampal neurons in response to BBG and glutamate (Glu).

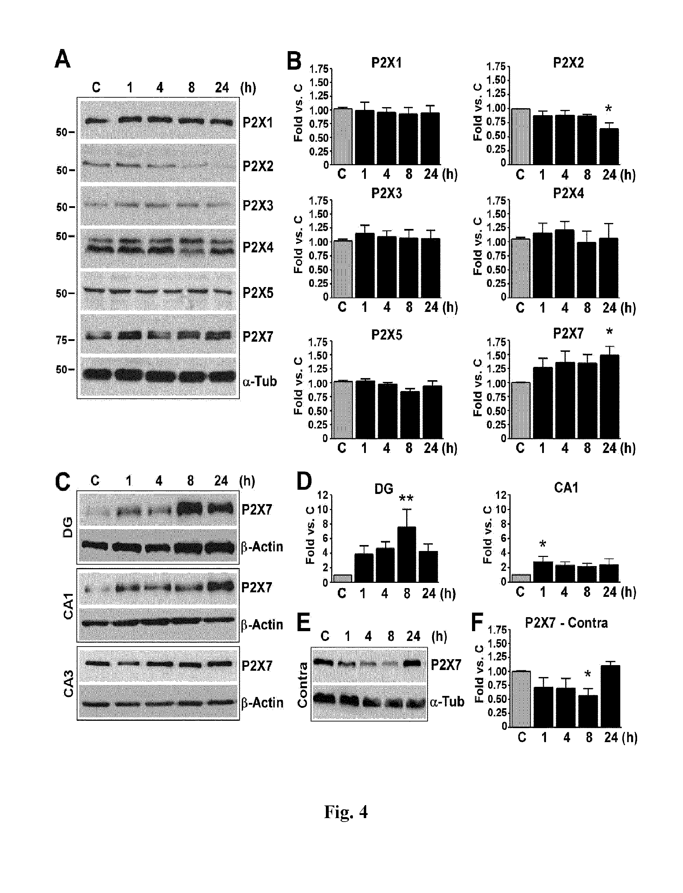

### Fig. 4: Altered P2XR expression after SE in hippocampus

**(A)** Representative western blots (*n* = 1 per lane) showing expression of different P2X receptor subtypes (P2XR1-5 and 7) after SE in whole ipsi-lateral hippocampus. α-Tubulin ("α-Tub" in the Figure) is shown as loading control. **(B)** Quantification of P2X receptor protein levels of whole ipsi-lateral hippocampus after SE, expressed as change fold versus the control (bars labeled as "C") (*n* = 4). **(C)** Representative western blots (*n* = 1 per lane) showing P2X7R levels in the different subfields of the ipsi-lateral hippocampus (CA3: cornu ammonis 3, CA1: cornu ammonis 1, and DG: dentate gyrus) after SE. β-Actin is shown as loading control. **(D)** Quantification of P2X7R protein levels of different ipsi-lateral hippocampal subfields after SE *(n* = 7-6). **(E)** Representative western blots *(n* = 1 per lane) showing P2X7R protein levels of the whole contra-lateral hippocampus. **(F)** Quantification of P2X7R protein levels in whole contra-lateral hippocampus showing significant down-regulation of the P2X7R at 8 h after SE. *n* = 4; *p < 0.05

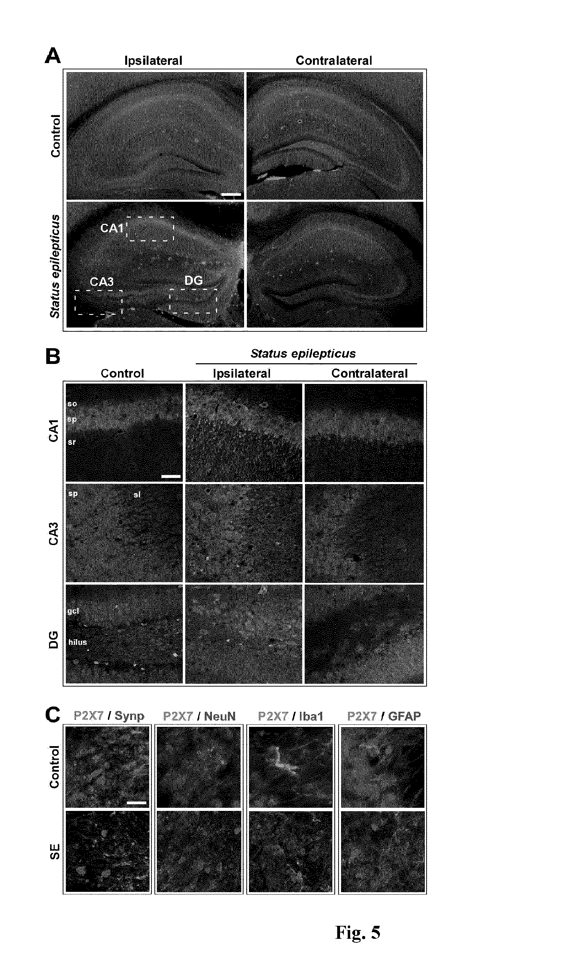

### Fig. 5. Hippocampal distribution and cell phenotype expressing P2X7.

**(A)** Hippocampal P2X7R staining (4 x lens) in control and after SE (24 h). P2X7R immunoreactivity is apparent within the main hippocampal subfields and increases ipsilaterally while appearing to decline in the contralateral side. (B) Double-label immunofluorescence staining (20 x lens) for P2X7R (green in the original) and NeuN (red in the original) in the main hippocampal subfields at 24 h. P2X7R is co-localized with NeuN in control conditions and after SE. (C) Double-label immunofluorescence staining for P2X7R (green in the original) and, synpatophysin (Syn), NeuN, Iba-1 or GFAP (red in the original) as indicated over the photographs, at 24 h: P2X7R signal appear colocalized with the signal corresponding to the other marker, except in the case of GFAP. Abbreviations: so, stratum oriens; sp, stratum pyramidal; sr, stratum radiatum; sl, stratum lucidum; gcl, granular cell layer. Scale in *A,* 300 µm, *B,* 80 µm, C 20 µm.

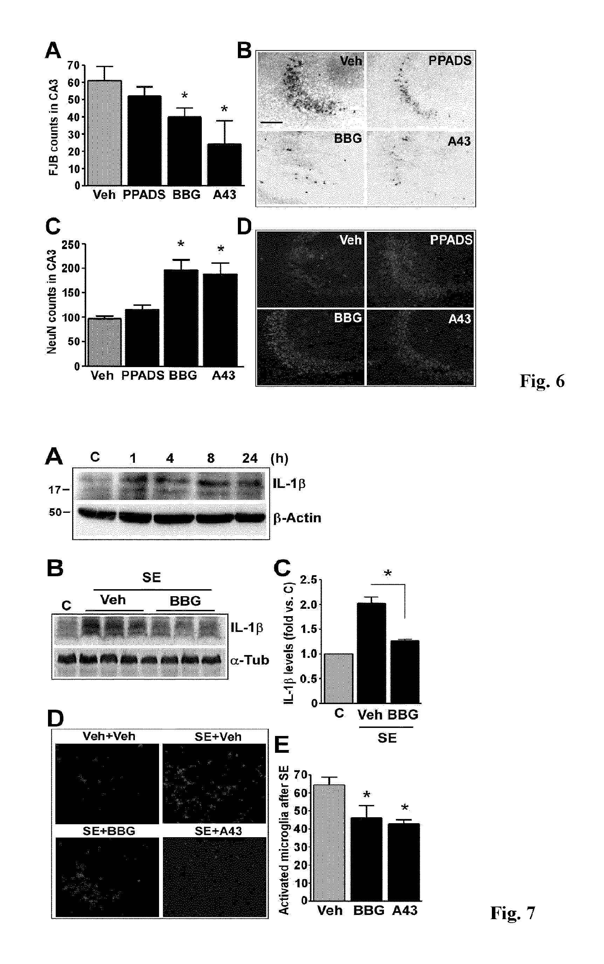

### Fig. 6: Pre-treatment with P2X7R antagonists protects against neuronal death during SE.

**(A)** FJB positive cell counts in the CA3 subfield of the ipsilateral hippocampus 24 h after SE. Injection of BBG and A-438079 (abbreviated A43) shortly before KA and immediately after lorazepam reduced cell death. **p* < 0.05 compared to Veh, vehicle. **(B)** Representative photomicrographs of FJB-stained CA3 neurons in the ipsilateral hippocampus at 24 h for each group. **(C)** Surviving neuron counts in the CA3 subfield 24 h after SE showing protective effect of BBG and A-438079. **(D)** Representative photomicrographs of NeuN-stained CA3 after SE. Note, improved survival of neurons in mice injected with A-438079 and BBG. Graphs, *n* = 5-12 per group. Scale bar in *A* is 180 µm

### Fig. 7: P2X7R antagonists block IL-1β and suppress microglia after SE

°**(A)** Representative western blot *(n* = 1 per lane) showing early and sustained increase in IL-1β protein levels after SE. β-Actin is included as a guide to protein loading. **(B)** Western blot (*n* = 1 per lane) showing SE-induced increase in IL-1β24 h after KA injection is blocked in animals injected with BBG. α-Tubulin is included as a loading control. **(C)** IL-1βprotein levels after SE showing reduction in BBG-treated mice *(n* = 3, **p* < 0.05). **(D)** Representative fluorescence Iba-1 immunostaining of microglia in the CA3 region of vehicle (Veh) and SE-treated mice showing the effect of BBG and A-438079. Note reduced microglia in A-438079- and BBG-treated animals after SE. Scale bar, 200 µm; **(E)** Activated microglia counts 24 h after SE in Veh, BBG and A-438079-injected mice *(n* = 12 per group); **p <* 0.05 compared to Veh.

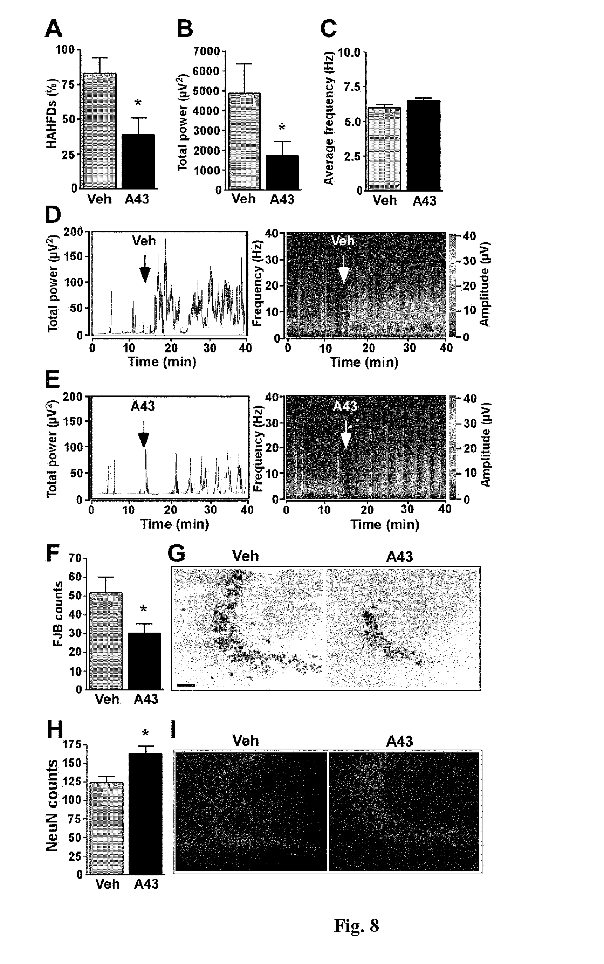

### Fig. 8: P2X7R inhibition after KA injection reduces seizure time during SE

**(A)** HAHFD (seizure) duration recorded after vehicle (Veh) or A-438079 injection (20 min after KA injection) until injection of anticonvulsant lorazepam (40 min after KA injection). **(B)** Total EEG power during seizures between groups. **(C)** Average frequency band range in each group. **(D)** Graph depicting typical power spectral density and adjacent spectrogram false color representation of spectral power against time and frequency during SE in vehicle-treated mice. **(E)** Power spectral density and adjacent spectrogram of spectral power against time and frequency during SE in an A-438079-injected animal. **(F)** FJB counts in the ipsilateral CA3 subfield of the hippocampus of mice injected with A-438079 15 min post-KA compared to vehicle-injected mice. **(G)** Representative photomicrographs of the CA3 showing less FJB-positive cells in A-438079 post-KA treated mice. **(H)** Surviving neuron counts in mice treated with vehicle or A-438079 after KA. **(I)** Representative photomicrographs of NeuN-stained CA3 at 24 h after SE for each group. Graphs are *n* = 10-15 per group; Scale bar in D is 100 µm. **p* < 0.05 compared to Veh.

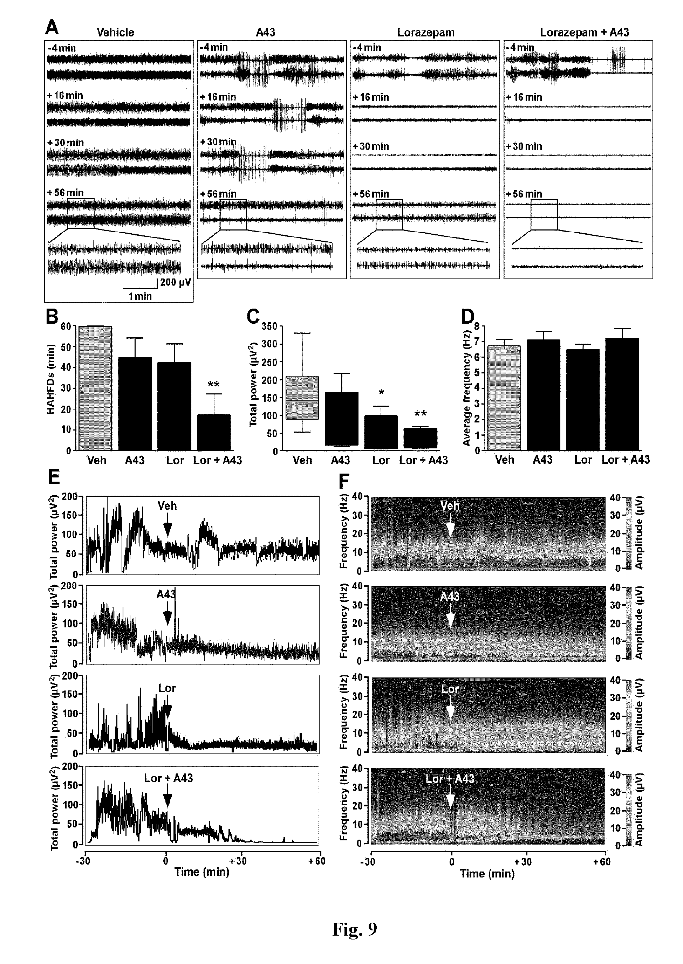

### Fig. 9: P2X7 inhibition potentiates the anticonvulsive effects of lorazepam once SE is refractory

**(A)** Representative EEG traces showing continuous electrographic epileptiform activity (HAHFDs) starting one hour after KA and over the subsequent 60 min. Note, SE was incompletely suppressed by lorazepam alone indicting partial refractoriness, but sustained cessation of spiking by lorazepam and A-438079 combination. **(B)** HAHFD duration in each group calculated once SE was continuous (1 h after KA). Lorazepam plus A-438079 resulted in significant seizure suppression. **(C)** Total power analysis for each group. Note, significant reduction in power in lorazepam (∼50%, **p* < 0.05 when compared to Veh) and lorazepam plus A-438079 group (∼70%, ***p* < 0.01 compared to Veh). **(D)** Mean band frequency was not different between groups. **(E)** Graphs (left) depicting typical power spectral density and (right) spectrogram false color representation of spectral power against time and frequency during SE showing the impact of each drug or combination. Note sustained seizure suppression is evident in the lorazepam plus A43 group. *n* = 5 - 7 per group.

### Fig, 10: P2X7 inhibition reduces seizure time in a neonatal rat model of SE

**(A)** Graph showing significant less amplitude in p10 rat pups treated 40 minutes after intraamygdala KA injection with intraperitoneal A-438079 when compared to vehicle injected p10 rat pups (n = 10 - 9) **(B)** Graph showing significant less total power in p10 rat pups treated 40 minutes after intraamygdala KA injection with A-438079 when compared to vehicle injected p10 rat pups (n= 10 - 9). **(C)** Power spectral density and adjacent spectrogram false color representation of spectral power against time and frequency during SE in vehicle (PBS) and A-438079 treated p10 rat pups. **(D)** Hippocampal P2X7R staining (20 x lens) after SE (24 h) to confirm presence of P2X7R in p10 rat pups. P2X7R immunoreactivity is apparent within the main hippocampal subfields (DG and CA3). Double-label immunofluorescence staining for P2X7R (green in the original) and NeuN (red in the original) reveal co-localization with the neuronal marker NeuN. Scale is 80 µm.

### DETAILED DESCRIPTION OF THE INVENTION

The present invention relates to the use of P2X7R antagonists, alone or in combination with benzodiazepines and/or any other GABA-based anti-convulsant such as a barbiturate, for the treatment of *status epilepticus* (SE) in general and, more particularly, for refractory *status epilepticus,* the situation reached when seizures burst become longer and longer until they become continuous, at which point benzodiazepines and other pharmacological treatments are ineffective to terminate seizures.

The present invention is supported by the findings disclosed in the present application, that are based *on in vivo* studies carried out with animal models of neonatal and adult *status epilepticus,* where the role of the ATP-activated P2X7 receptor has been studied. The assays demonstrate that experimental SE in mice rapidly up-regulated P2X7 receptor (P2X7R) levels in the hippocampus and support a role for P2X7R underlying the patho-mechanisms of SE.

As described in the Examples set forth below, central brain injection of a drug which blocks the P2X7 receptor significantly reduced seizure duration in mice when given just before or after triggering SE and potently protected the brain from seizure-damage. In mice given a P2X7 antagonist, the majority of animals never progressed to continuous seizures and, in some cases, became seizure-free. The Examples also provide evidence that these animals displayed significantly less brain damage than the vehicle-treated animals.

Moreover, adjunctive delivery of P2X7R antagonists with lorazepam was also effective at curtailing pharmacoresistant seizures an hour after SE initiation. Thus, P2X7R activation may contribute to development and maintenance of SE. Administration of P2X7R antagonist, preferably a selective P2X7R antagonist, alone or as adjunctive treatment with benzodiazepines or barbiturates, can be used to significantly shorten and, in some cases, fully terminate *status epilepticus,* even when it has become pharmacorefractory. Besides, the administration of P2X7R antagonists has a positive effect in preventing or ameliorating the brain damage associated with *status epilepticus.*

What is more, intraperitoneal injection of the same P2X7R antagonist, A-438079, to a rat model of neonatal seizures (that could be classified as neonatal *status epilepticus,* assuming that it exists), strongly reduced seizures, what serves to establish that P2X7R antagonist can be used to significantly reduced seizures in conditions of prolonged neonatal seizures. The mentioned assays also demonstrate that the intraperitoneal or intravenous routes are suitable routes that can be used for the administration of P2X7R antagonists capable to cross the blood brain barrier when it is sought an amelioration of prolonged seizures, even in neonatal models.

This is a surprising effect, taking into account the prior disclosures of the same technical field. P2X7R have previously been implicated in the pathophysiology of neurological and neurodegenerative conditions, and a potential role for P2X7R in epilepsy had been already suggested (Vianna et al., 2002; Diaz-Hernandez et al., 2009). However, there was no specific evidence to date proving functional involvement in seizures *in vivo,* nor any *in vivo* data suggesting that P2X7R antagonists could affect seizures in living animals, because no previous studies have even explored possible direct seizure-suppressive properties of P2X7R antagonists. As discussed above, not even the previously proposed applications of P2X7R antagonists for the treatment of neurodegenerative diseases had been experimentally demonstrated, the data about effects on nociception and IL-1 release being practically the only experimental results available about the possible therapeutic utility of P2X7R antagonists. Indeed, a protective effect of P2X7R-targeting drugs on astrocyte survival was recently reported (Kim et al., 2010), but the effect was observed after SE, not having been suggested that P2X7R antagonists could be used to disrupt prolonged seizures (*status epilepticus),* even when they have become pharmacologically refractory.

The major finding of the present study was that P2X7R blockers given either shortly before or after onset of SE potently suppressed seizures. The experiments carried out with two animal models have shown that, in a pre-treatment regime, pharmacological inhibition of the P2X7R suppressed seizures during SE, and significantly reduced microglial activation, levels of the pro-convulsive cytokine interleukin-1β and neuronal death. P2X7R antagonist was also seizure-suppressive and neuroprotective when injected shortly after induction of SE.

The results have been obtained with two focal-onset SE models which reliably propagate seizures to the hippocampus (Murphy et al., 2010), obtained by intra-amygdala microinjection of the glutamate agonist kainic acid (KA). In this model, seizures are propagated to the hippocampus via the perforant pathway, producing hippocampocentric seizures and consistent CA3 and hilar damage, thereby avoiding direct neurotoxic effects on the hippocampus and the limited recruitment of the hippocampus during SE evoked by systemic pilocarpine and KA (Sloviter et al., 2007). These are the first *in vivo* data demonstrating anticonvulsant effects of targeting this receptor, and have been obtained both in the mouse model of adult SE and in the rat model of neonatal SE. The conclusion is supported by complementary lines of evidence. First, seizures could be suppressed by BBG and A-438079, both selective P2X7R antagonists at the doses used, and seizures were potentiated by BzATP, a P2X7R agonist (Jiang et al., 2000; McGaraughty et al., 2007). A neutralizing P2X7R antibody also reduced SE. The non-selective P2XR antagonist PPADS did not reduce seizures, perhaps because blocking all P2X-mediated signaling would have reduced the inhibitory effects mediated by P2X2R (Khakh et al., 2003; Khakh and North, 2006).

P2X7R inhibition did not, however, completely terminate SE. This was not unexpected because glutamate-gated channels independently contribute to both the development and maintenance of SE (Wasterlain and Chen, 2008). While P2X7R antagonists are probably not superior to glutamate receptor antagonists at stopping SE (Mazarati and Wasterlain, 1999), their restricted CNS distribution and activation under more pathophysiologic levels of neuronal activity make them more attractive targets which may avoid some of the adverse side-effects associated with glutamate-blocking drugs (Burnstock, 2008; Green and Cote, 2009). The actual mechanism of the observed anticonvulsant effect is uncertain. Without wishing to be bound by a theory, it might speculated that it may arise from preventing pre-synaptic P2X7R-mediated enhancement of glutamate release (or another neurotransmitter), or post-synaptic potentiation of excitatory currents (Pankratov et al., 1998; Deuchars et al., 2001; Vianna et al., 2002; Papp et al., 2004; Diaz-Hernandez et al., 2009; Cho et al., 2010). The pro-convulsive influence of P2X7R differs markedly from responses mediated by P2YR and certain hippocampus-expressed P2XR where inhibition is the major consequence of their activation, contributing to a brake on excessive excitation (Khakh and North, 2006). Taken together, the data disclosed in the present application support a P2X7R contribution to seizures which can be pharmacologically targeted to reduce SE.

The immunohistochemical and western blot analyses described in Examples of the present invention support both neuronal and microglial expression of P2X7R in the hippocampus and show a selective increase in P2X7R levels after SE. This is in agreement with other work demonstrating neuronal expression of P2X7R in the CNS, including the hippocampus, and an increase after prolonged seizures (Deuchars et al., 2001; Vianna et al., 2002; Atkinson et al., 2004; Sperlagh et al., 2006; Yu et al., 2008). Notably, somal P2X7R staining declined in CA3 neurons after SE and emerged in a pattern that co-localized with a pre-synaptic compartment, presumably the terminals of the mossy fibers, patterns that have been reported previously (Miras-Portugal et al., 2003; Atkinson et al., 2004). Such pre-synaptic enrichment in terminals may influence transmitter release (Gualix et al., 2003; Papp et al., 2004; Cho et al., 2010), and contribute to the changing sensitivity of the brain to late versus early administration of P2X7R antagonists. Thus, P2X7R, like GABA_{A} and NMDA receptors (Naylor et al., 2005; Wasterlain et al., 2009), undergo dramatic cellular re-distribution during SE. Of note, expression of the P2X2R decreased after SE. This could have pro-convulsive consequences because P2X2R activation promotes inhibitory drive in the hippocampus (Khakh et al., 2003).

P2X7R are also expressed by non-neuronal cell types in the brain. Here, they contribute to microglia activation and subsequent release of cytokines, including IL-1β (Ferrari et al., 1996; Labrousse et al., 2009; Monif et al., 2009). Indeed, caspase-1 the enzyme responsible for IL-1β production, is activated downstream of P2X7R (Ferrari et al., 1999). The data provided in the present application confirm that P2X7R expression increases within microglia after SE, as reported by others (Rappold et al., 2006; Avignone et al., 2008), and also show P2X7R antagonists reduced hippocampal microglia proliferation and IL-1β levels after SE. This may also be a mechanism contributing to seizure suppression since increased hippocampal IL-1β levels prolong seizures during SE (Vezzani et al., 1999) and genetic deletion of caspase-1 is anticonvulsant *in vivo* (Ravizza et al., 2006). Thus, attenuation of adverse microglia-mediated neuroinflammation may be a secondary mechanism underlying the anticonvulsive effects of P2X7R antagonists during SE.

In the present study, all mice pre-treated or post-treated shortly after seizure onset with inhibitors of P2X7R displayed significantly reduced hippocampal damage. This is likely a direct consequence of the shorter duration of SE, since seizure-damage is broadly related to seizure time, including in the present model (Fujikawa, 1996; Araki et al., 2002; Murphy et al., 2007). A direct anti-apoptotic effect of blocking P2X7R is also possible, since blocking neuronal P2X7R *in vivo* has been reported to be anti-apoptotic (Diaz-Hernandez et al., 2009). Notably, mice deficient in certain core proapoptotic signaling molecules are protected against seizure-induced neuronal death in the model used here (Engel et al., 2010; Murphy et al., 2010).

In accordance with all these results, a first aspect of the invention relates to an antagonist of P2X7 receptor, for the treatment of *status epilepticus.* The antagonist can be used alone or in combination with an anti-convulsant, particularly a benzodiazepine.

In principle, any P2X7R antagonist can be used, including the traditional ones such as BBG or KN-62, as the positive effect of P2X7R antagonism in SE might be linked to preventing pre-synaptic P2X7R-mediated enhancement of glutamate release (or another neurotransmitter), or post-synaptic potentiation of excitatory currents. Indeed, the Examples of the invention show that seizures could be suppressed by different P2X7R antagonists, such as BBG and A-438079, and a neutralizing P2X7R antibody also reduced SE. However, the fact that PPADS, a non-selective P2XR antagonist did not reduce seizures, makes preferable the use of selective antagonists.

A possibility for the election of the antagonist is to resort to the series of compounds, based in a common structure, developed in the last years in order to find more selective antagonists, such as aryl carbohydrazides like diarylimidazolines, cyanoguanidines like the compound known as A-740003 ((*N*-(1-{[cyanoimino)(5-quinolinylamino)methyl]amino}-2,2-dimethylpropyl)-2-(3,4-dimethoxyphenyl)acetamide) or disubstituted tetrazoles like the compound known as A-438079 (3-((5-(2,3-dichlorophenyl)-1*H*-tetrazol-1-yl)methylpyridine), which is an antagonist used in the assays of the Examples of the present application. Then, in a possible embodiment, the antagonist is one of the compounds of the family of disubstituted tetrazoles described in US Patent Application N. 2006/0211739, the family of A-438079, or other compounds of the same family developed later, such as the compound known as A-839977 (1-(2,3-dichlorophenyl)-N-[2-(pyridin-2-yloxy)benzyl]-1H-tetrazol-5-amine), whose structural formula is as follows:

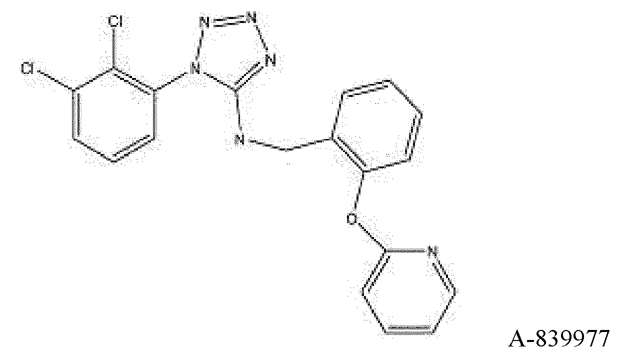

Then, in a possible embodiment, the antagonist is selected from the disubstituted tetrazoles of the Formula I, 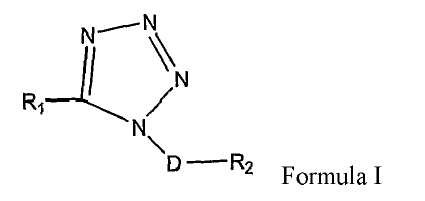 wherein,
R1 is selected of the groups consisting of cycloalkenyl, cycloalkyl, Heterocyclo, aryl, Heteroaryl, arylalkyl and Heteroarylalkyl; wherein each R1 is substituted with 0, 1, 2, 3, 4 or 5 sustituents which are independently selected from the group consisting of alkyl, alkenyl, alkinyl, nitro, cyano, Halo, -OR_{C}, -O(CO)R_{c}, -OC(O)OR_{c}, -OS(O)₂R_{C}, - SR_{C}, -S(O)R_{c}, -S(O)₂R_{C}, -S(O)₂OR_{c}, -S(O)₂NR_{c}R_{d} , -NR_{c}R_{d}, -N(R_{d})C(O)OR_{c}, - N(R_{d})C(O)NR_{c}R_{d}, -N (R_{d})S(O)₂NR_{c}P_{d}, -C(O)R_{c}, -C(O)OR_{c}, -C(O)NR_{c}R_{d}, haloalkyl, cianoalkyl, nitroalkyl, -alkylOR_{c}, -O(CO)R_{c}, -alkylOC(O)OR_{C}, -alkylOS(O)₂R_{C}, alkylSR_{c}-, -alkylS(O)R_{c}, -alkylS(O)₂R_{C}, -alkylS(O)₂OR_{c}, -alkylS(O)₂NR_{c}R_{d}, alkylNR_{c}R_{d}, -alkylN(R_{d})C(O)OR_{C}, -alkylN(R_{d})C(O)NR_{c}R_{d}, -alkylN(R_{d})S(O)₂NR_{c}R_{d}, alkylC(O)R_{c}, -alkylC(O)OR_{C}, -alkylc(O)NR_{c}R_{d} and R3; provided that, when R1 is arylalkyl or Heteroarylalkyl, D is a bond;
R2 is selected of the groups consisting of cycloalkyl, cycloalkenyl, Heterocyclo, aryl and Heteroaryl; wherein each R2 is substituted with 0, 1, 2, 3, 4 or 5 sustituents which are independently selected from the group consisting of alkyl, alkenyl, alkinyl, nitro, cyano, Halo, -OR_{C}, -O (CO)R_{c}, -OC(O)OR_{c}, -OS(O)₂R_{C}, -SR_{C}, -S(O)R_{c}, - S(O)₂R_{C}, -S(O)_{2O}R_{c}, -S(O)₂NR_{c}R_{d} , -NR_{c}R_{d}, -N(R_{d})C(O)OR_{c}, -N(R_{d})C(O)NR_{c}R_{d}, - N(R_{d})S(O)₂NR_{c}R_{d}, -C(O)R_{c}, -C(O)OR_{c}, -C(O)NR_{c}R_{d}, Haloalkyl, cyanoalkyl, nitroalkyl, -alkylR_{c}, -O(CO)R_{c}, -alkylC(O)OR_{C}, -alkylS(O)₂R_{C}, alkylSR_{c}, -alkylS(O)R_{c},-alkylS(O)₂R_{C},-alkylS(O)₂OR_{c},-alkylS(O)₂NR_{c}R_{d}, alkylNR_{c}R_{d}, -alkylN(R_{d})C(O)OR_{C}, - alkyllN(R_{d})C(O)NR_{c}R_{d}, -alkylN(R_{d})S(O)₂NR_{c}R_{d}, alkylC(O)R_{c}, -alkylC(O)OR_{C}, - alkylC(O)NR_{c}R_{d} and R3;
R3 is selected of the group consisting of cycloalkyl, cycloalkenyl, Heterocyclo, aryl and Heteroaryl; wherein each R3 is substituted with 0, 1, 2, 3, 4 or 5 substituents which are independently selected from the group consisting of alkyl, alkenyl, alkinyl, nitro, cyano, Halo, formyl, Hydroxy, alkoxy, Haloalkoxy, -OC(O)alkyl, -S(O)₂alkyl, - S(O)₂NH₂, -S(O)₂N(H)(alkyl), -S(O)₂N(alkyl)₂, -NH₂, -N(H)(alkyl), -N(alkyl)₂, - C(O)(alkyl), -C(O)(OH), -C(O)(Oalkyl), -C(O)NH₂, -C(O)N(H)(alkyl), -C(O)N(alkyl)₂, Haloalkyl, formylalkyl, cyanoalkyl, nitroalkyl, Hydroxyalkyl, alkoxyalkyl, Haloalkoxyalkyl, -alkylC(O)alkyl, -alkyl-S(O)₂alkyl, -alkyl-S(O)₂NH₂, -alkylS(O)₂N(H)(alkyl), -alkyl-S(O)₂N(alkyl)₂, -alkyl-NH₂, -alkyl-N(H)(alkyl), -alkyl-N(alkyl)₂, -alkyl-C(O)(alkyl),- alkyl-C(O)(OH), -alkyl-C(O)(Oalkyl), -alkyl-C(O)NH₂, - alkyl-C(O)N(H)(alkyl), and -alkyl-C(O)N(alkyl)₂; and
R_{c} y R_{d}, in each occurrence, are independently selected from the group consisting of hydrogen, alkyl, haloalkyl, aryl, heteroaryl, arylalkyl and heteroarylalkyl; wherein the aryl, heteroaryl, aryl of the arylalkyl moiety and heteroaryl of the heteroarylalkyl moieties are independently substituted with 0, 1, 2 or 3 substituents which are independently selected from the group consisting of alkyl, halo, haloalkyl, hydroxy, hydroxyalkyl and alkoxialkyl.

In a preferred embodiment, the particular disubstituted tetrazole is one of the compounds whose activity was studied by Nelson et al. (Nelson et al. 2006) or a commercial compound with known activity such as A-839977. Then, in that particular embodiment, the antagonist is selected from one of the following groups:
a) the disubstituted tetrazoles of Formula II 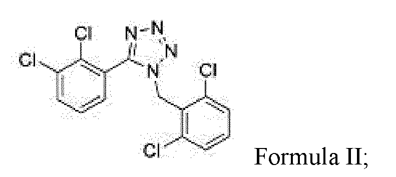
b) the substituted tetrazoles of Formula III 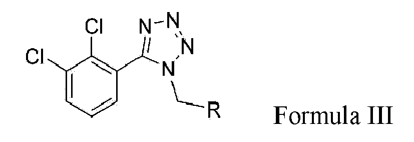 wherein R is selected from the group of C₆H₅, 2-CH₃-C₆H₆, 2-pyridyl, 3-pyridiyl, 4-pyridyl, 2-CH₃-3-pyridyl, 3-pyridinylmethyl, 2,4-(CH₃)₂-5-thiazolyl, 3,5-(CH₃)₂-4-isoxazolyl;
c) the substituted tetrazoles of Formula IV 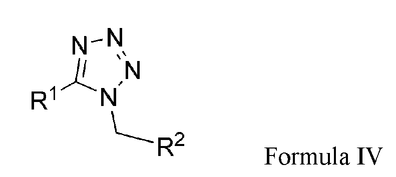 wherein,
   R² and R¹ are selected so that
   when R² is 3-pyridyl, R¹ is selected from the group of C₆H₅, 2-Cl-C₆H₄, 3-Cl-C₆H₄, 2,5-Cl₂-C₆H₃, 3,4-Cl₂-C₆H₃, 2-CF₃-3-F-C₆H₃, 2-Cl-3-CF₃-C₆H₃, 2-F-3-CF₃-C₆H₃, 2,3-Cl₂-4-F-C₆H₂, 2,3,4-Cl₃- C₆H₂,
   when R² is C₆H₅, R¹ is selected from the group of 4-pyridyl, 5-quinolyl, 8-quinolyl, 2,3-Cl₂-4-pyrrolidinyl-C₆H₂,
   when R² is 2-CH₃-C₆H, R¹ is 2,3-(CH3)2-C₆H_{3,}
   when R² 2-Cl-C₆H₄, R¹ is 2,3-(OCH₃)₂-C₆H₃;
d) the substituted tetrazoles of Formula V, 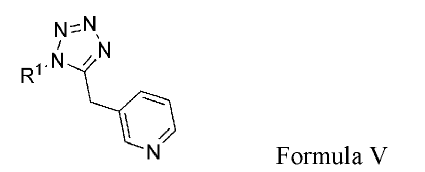 wherein R¹ is selected from the group of 2,3-Cl₂-C₆H₃, 2,3-Cl₂-4-F-C₆H₂, 2-Cl-3-CF₃-C₆H₃, 2-Cl-3-CF₃-4-F-C₆H₂;
e) 1-(2,3-dichlorophenyl)-N-[2-(pyridin-2-yloxy)benzyl]-1H-tetrazol-5-amine (A-839977);
or any pharmaceutically acceptable salt thereof.

A-438079, one of the P2X7R antagonists used in the Examples of the present application, belongs to the group of compounds of Formula V, wherein R¹ is 2,3-Cl₂-C₆H₃.

The results have been obtained with two animal models: one model (mouse model) of adult *status epilepticus* and a second model of neonatal *status epilepticus.* The animals used for the study of neonatal *status epilepticus* were 10 day old (P10) rat pups (age equivalent to human neonates) (Dunleavy et al., 2010). In both cases, the P2X7R antagonist used strongly reduced seizures. This a support for the use of antagonists of P2X7 receptor for the treatment of *status epilepticus* in general, both in adults as well as in individuals of lower age such as children, infants and neonates.

In the mouse model, the drugs were administered directly to the brain, by intracerebroventricular (i.c.v.) injections. In the second model (neonatal rat model), the P2X7R antagonist was administered intraperitoneally. Thus, in order to have a route of administration more suitable and accessible for human beings than the intracerebroventricular injection, advantage can be taken of the fact that some P2X7R antagonists are capable to cross the blood brain barrier after systemic administration and exert a positive effect in the brain, as demonstrated in International Application WO2010/018280 and confirmed by the assays disclosed in Example 6 of the present application. Therefore, among all the possible antagonists, those capable to cross the blood brain barrier are preferred. Some examples of such antagonist are Brilliant Blue G (BBG), KN-62, A-438079 or any pharmaceutically acceptable solvate thereof (such as hydrochloride), or any pharmaceutically acceptable salt of decavanadate ((V₁₀O₂₈)⁶⁻).

As shown in Examples of the present application, the majority of animals treated with a P2X7R antagonist never progressed to continuous seizures and, in some cases, became seizure-free. Then, the P2X7R antagonist can be administered alone not only with the aim of decreasing the severity and/or the duration of SE, but even to prevent the progression to the state where the seizures become refractory to current treatment with benzodiazepines, the situation known as refractory *status epilepticus.*

P2X7R antagonists can be administered alone, or in combination with any other anti-convulsant. This second option is preferred when the total interruption of a seizure is sought. The anti-convulsant can be a benzodiazepine, as they are the most common frontline treatment for status epilepticus. Lorazepam, the most commonly used benzodiazepine for the treatment of seizures, is also the preferred benzodiazepine for the purposes of the invention. Any other anti-convulsant or any other drug approved for the treatment of seizures can be also used, such as a barbiturate, or a currently in-trial medication such as bumetanide (3-butylamino-4-phenoxy-5-sulfamoyl-benzoic acid). Particularly in the case when the individual to be treated is a neonate, an infant or a child up to five years of age, it must be taken into account if the selected drug is approved for the treatment of seizures in individuals of that age.

The individual administration of a P2X7R antagonist is not effective to interrupt refractory status epilepticus. However, the P2X7R antagonist was capable to disrupt pharmacologically refractory *status epilepticus* when it is administered in adjunction with a GABA-based convulsant, such as a benzodiazepine like lorazepam, is particularly surprising. The combined administration of an antagonist of P2X7R (namely A-438079 in the Examples of the present application) and a benzodiazepine (lorazepam in Examples of the present application) give rise to the effective interruption of the seizures that, up to know, where refractory to the pharmacological treatment, particularly the treatment with GABA-based anticonvulsants, significantly decreasing the duration and the potency of such seizures. Example 5 in particular discloses how the P2X7R antagonist was able to potentiate the effect of lorazepam once seizures became intractable (as evinced by the weak anti-seizure effect of lorazepam alone). This is a very surprising effect, because it means an unexpected synergistic interaction of both drugs, particularly surprising taking into account that neither the individual administration of lorazepam nor the individual administration of the P2X7R antagonist A-438079 are effective to interrupt refractory seizures, as demonstrated by the authors of the present invention. Then, the invention is particularly useful in providing a solution to the need of alternative or adjunctive polytherapy for pharmacoresistant SE when GABA-based drugs such as benzodiazepines lose efficacy as seizures become protracted and continuous. Even when the internalization of the GABA_{A} receptor, its desensitization and/or its downregulation has occurred, administration of P2X7R antagonists makes possible the interruption of seizures otherwise resistant to benzodiazepines, surprisingly, upon their administration in adjunction with a compound whose mechanism of action is linked to the interaction with GABA_{A} receptors and that, even in the presence of P2X7R antagonist, should remain practically ineffective: a benzodiazepine such as lorazepam. Without wishing to be bound by any theory, the authors of the present invention hypothesize that the synergistic effect of P2X7R antagonism with lorazepam on pharmacoresistant seizures could have a relationship with ATP release during SE, giving rise to an activation of P2X7R that effectively reduces GABAergic inhibition. The fact that delayed administration of A-438079 alone is not as effective at stopping seizures as when injected early during SE might be the result of hippocampal subfield changes, possibly via trafficking, in the population of targeatable P2X7R (Murrell-Lagnado and Qureshi, 2008). Regardless, P2X7R antagonists, as demonstrated by the assays disclose in the present application, appear as suitable adjunctive anticonvulsive treatment in combination with benzodiazepines for refractory SE.

Accordingly, in a preferred embodiment of the invention, the status epilepticus to treat is refractory status epilepticus. In this particular case, the combined administration with a benzodiazepine, especially lorazepam, is preferred. In such specific embodiment, the administration of A-438079 as P2X7R antagonist, as in some Examples of the present application, is specially preferred.

In a second aspect, the invention refers to a method of use of an antagonist for treating the seizure in the *status epilepticus* in an animal, which method comprises administering to said animal an effective amount of the antagonist of P2X7 receptor or a pharmaceutically acceptable salt thereof.

Example 1 shows that the administration of a P2X7R antagonist prior to SE induction significantly reduced seizure time during SE. Very interestingly for its therapeutic utility, as shown in Example 4, treatment with P2X7R antagonists during SE also reduces seizure time and hippocampal damage. This indicates a possible utility of P2X7R antagonists to reduce the severity and duration of seizure even when they are administered prior to the seizure. Then, the P2X7R antagonist can be administered to the animal prior to, contemporaneous with, or subsequent to start of the seizure.

The antagonist can be administered to the animal alone; another alternative is the administration in combination with another drug, preferably a benzodiazepine. As previously commented, the other drug can also be any other anti-epileptic drug, such as a barbiturate, or currently in-trial medication such as bumetanide. Specially in the case of infants and children, and very specially in the case of neonates, attention should be given to check if the intended adjunctive drug has been approved for the treatment of individuals of that age. The antagonist can be administered prior to, simultaneously to, or subsequent to the administration of a benzodiazepine or the other anti-epileptic drug.

The status epilepticus can be in the pharmacorefractory state (refractory status epilepticus); in this case, the administration in adjunction with a benzodiazepine is preferred, because P2X7R antagonists alone are not able to terminate seizures, but they potentiate the ability of benzodiazepines such as lorazepam to achieve the total cessation of the seizure. As in Example 5, the simultaneous administration, or with a short separation in time, of benzodiazepine and P2X7R antagonist is preferred.

The administration of the antagonist of the P2X7 receptor can be directly to the central nervous system. As the preferred animals to treat are human beings, other routes of administration are preferred. Parenteral administration (e.g., intraperitoneally or intravenously, as indicated below); in that case, antagonists of P2X7 capable to cross the blood brain barrier must be used.

As mentioned above, the preferred animals to treat are human beings. They can be adults, or children, infants or neonates.

As used in the present application, *status epilepticus* is defined as seizures lasting longer than 5 min in a human adult, or more than 12 min in a child (age lower than 13 years). In the case of neonates, *status epilepticus* is defined as continuous seizure activity for at least 30 minutes or recurrent seizures lasting a total of more than 30 minutes without definite return to the baseline neurologic condition of the newborn between seizures; then, it is a case of prolonged neonatal seizures. Refractory *status epilepticus* is defined as continued seizures after two or three anti-epileptic drugs have failed. Particularly, refractory *status epilepticus* does not respond to first-line benzodiazepines lorazepam or diazepam and/or to second-line antiepileptic drugs (phenytoin/fosphenytoin, phenobarbital or valproate).

As used in the present application, a human neonate is in the first month of its life. An infant is a child in the earliest period of life, between the ages of 1 month and 12 months. Children, in turn, are younger than thirteen years old. For the purpose of the present invention, adults are individuals older than twelve. Anyway, there is no evidence showing that children would show a different response to the invention than adults, and it could be considered in the same group.

As used in the present application, two drugs are administered in combination when they are administered as a part of the same schedule of treatment of a condition in a particular individual. Then, it is not necessary that they are administered simultaneously. Similarly, two drugs are administered in adjunction when one is added two the other as a part of the same schedule of treatment, the first one being considered a subordinate object that helps the first one to exert its action. Although they are not necessary administered together and/or simultaneously, a short difference between the time of administration (no longer than 30 minutes) is expected.

As used in the present application, any mentioned compound intended to be administered to a subject can be administered in the form of any pharmaceutically acceptable salt or solvate thereof. Thus, for instance, the P2X7R antagonist A-438079 can be administered as such or, for instance, as hydrochloride, one of the commercial forms of the same. The P2X7R will preferably be administered in the form of a pharmaceutical composition, in combination with a pharmaceutically acceptable carriers, adjuvants, diluents, vehicles, or combinations thereof. As used herein, pharmaceutically acceptable means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The compositions containing P2X7R antagonists can be formulated in a conventional manner using one or more of the aforementioned pharmaceutically acceptable carriers. The final composition will depend on the specific P2X7R antagonist selected and properties such as its solubility. Thus, for the purposes of the present invention, the P2X7R antagonist or its pharmaeutically acceptable salt or solvate, may be administered to humans and other mammals in solid or liquid form, orally, rectally, parenterally, intracistemally, intravaginally, topically (as by powders, ointments, drops, inhalants, spray, transdermal patch, and the like), or bucally. The preferred route, as previously mentioned, is the parenteral route. The term "parenterally," as used herein, refers to modes of administration which include intravenous, intramuscular, intraperitoneal, intrastemal, subcutaneous, intraarticular injection and infusion.

The compositions with P2X7R antagonists prepared for parenteral injection usuful for the purposes of the present invention comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity may be maintained, for example, by the use of a coating such as lecithin, by the maintenance of the required particle size in the case of dispersions, and by the use of surfactants.

These compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Suspensions, in addition to the active compounds, may contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof

If desired, and for more effective distribution, the compounds of the present invention can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

The active compounds can also be in micro-encapsulated form, if appropriate, with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound can be admixed with at least one inert diluent such as sucrose, lactose, or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of such composition that they release the active ingredient(s) only, or preferentially, in a certain part of the intestinal tract in a delayed manner. Examples of embedding compositions that can be used include polymeric substances and waxes.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic, parenterally acceptable diluent or solvent such as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin); f) absorption accelerators such as quaternary ammonium compounds; g) wetting agents such as cetyl alcohol and glycerol monostearate;) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract in a delayed manner. Examples of embedding compositions that can be used include polymeric substances and waxes.

Compositions for rectal or vaginal administration are preferably suppositories which can be prepared by mixing the P2X7 antagonist with suitable non-irritating excipients or carriers such as cocoa butter, polyethylene glycol or a suppository wax which are solid at ambient temperature but liquid at body temperature and therefore melt in the rectum or vaginal cavity and release the active compound.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chloroftuorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The P2X7 antagonist may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used. The present compositions in liposome form may contain, in addition to the compounds of the present invention, stabilizers, preservatives, excipients, and the like. The preferred lipids are the natural and synthetic phospholipids and phosphatidylcholines (lecithins) used separately or together. Methods to form liposomes are known in the art. See, for example, Prescott, Ed., Methods in Cell Biology, Volume XTV, Academic Press, New York, N. Y., (1976), p 33 et seq.

The phrase "therapeutically effective amount" or simply "effective amount" means a sufficient amount of the P2X7R antagonist to decrease seizure time and/or power. When the P2X7R antagonist is administered parenterally, it must taken into account that the compound must exert its activity in the brain; then administered amount should enough to achieve that a therapeutically effective amount reaches the brain. The specific therapeutically effective dose level for any particular patient will depend upon a variety of factors including the severity of the seizures, if the situation can be considered having reached the state of refractory status epilepticus (wherein the possible additional drugs administered should also be taken into account), the activity of the specific P2X7R antagonist employed; the specific composition employed; the age, body weight, general health, sex and diet of the patient; the time of administration, route of administration, drugs used in combination or coincidental with the specific compound employed; and other factors well known in the medical arts.

The invention will be explained in more detail by means of the Examples and Figures of the present application.

### EXAMPLES

The following Examples were carried out using the following techniques, compounds and experimental animals:

### - Seizure models

All animal experiments were performed in accordance with the principles of the European Communities Council Directive (86/609/EEC) and National Institute of Health's Guide for the Care and Use of Laboratory Animals. Procedures were approved by the Research Ethics Committee of the Royal College of Surgeons in Ireland. Procedures were undertaken as previously described (Araki et al., 2002; Engel et al., 2010).

Adult male C57BL/6 mice (20-25 g) were obtained from Harlan UK (Blackthorn, UK). Mice were first anesthetized using isoflurane (3-5%) and maintained normothermic by means of a feedback-controlled heat blanket (Harvard Apparatus Ltd, Kent, UK). Mice were next placed in a stereotaxic frame and three partial craniectomies performed to affix cortical skull-mounted EEG electrodes (Bilaney Consultants Ltd, Sevenoaks, UK). EEG was recorded using a Grass Comet XL digital EEG (Medivent Ltd, Lucan, Ireland). A guide cannula was affixed (coordinates from Bregma: AP = 0.94; L = 2.85 mm) and the assembly fixed in place with dental cement. Anesthesia was then discontinued and freely moving mice were placed inside a clear perspex recording chamber. EEG recordings were commenced and after establishing baseline EEG for a few minutes, an injection cannula was lowered through the guide cannula for injection of kainic acid (KA) (Ocean Produce International, Nova Scotia, Canada) into the basolateral amygdala nucleus (0.3 µg in 0.2 µl phosphate-buffered saline). Non-seizure control mice underwent the same surgical procedure but received 0.2 µl intra-amygdala vehicle. Mice were killed at different time-points (1, 4, 8 or 24 h) later and perfused with saline to remove intravascular blood components. Brains were either flash-frozen whole in 2-methylbutane at 30°C or perfused with 4% PFA for histopathology, or dissected on ice to obtain whole hippocampus or microdissected to obtain the different hippocampal subfields (CA1, DG and CA3).

10 day old Sprague Dawley rat pups were obtained from Harlan UK Limited (Blackthorn, UK). Rat pups were equipped with skull surface EEG and given intra-amygdala microinjection of kainic acid (2 µg) via an implanted guide cannula. P10 rat pups develop SE shortly after KA injection. Seizures in the model are propagated to the hippocampus and surface EEG recordings have previously shown a transition to low amplitude high frequency EEG, developing into high amplitude high frequency epileptiform EEG seizures. The pups display behaviour including initial masticatory movements and salivation, developing to periods of facial and forelimb myoclonus and progressing to the most commonly observed behaviour of wild running, loss of posture and swimming motions. The high amplitude high frequency spiking is sustained and corresponds to wild running and swimming behaviours during recordings 1-2 h after KA. By 4 hours, continuous seizures have abated but the EEG continues to display intermittent lower-amplitude high frequency spiking and high amplitude low frequency spikes even after 24 h. The pathological consequences of neonatal seizures in the model include hippocampal cell death and the animals later display unilateral hippocampal sclerosis.

### - Quantification of EEG

Digitized EEG recordings were analyzed off-line using manual assessment and automated software. The occurrence of high amplitude high frequency spiking during seizures is synonymous with injury-causing electrographic seizures during SE (also termed type IV electrographic seizures) (Lowenstein et al., 1991; Henshall et al., 2000). The duration of high-frequency (>5 Hz) high-amplitude (>2× baseline) polyspike discharges of ≥5 s duration (HAHFDs) was counted by a reviewer blind to treatment. Further EEG analysis was performed by uploading EEG into Labchart7 software (ADInstruments) to calculate the power spectral density (total power, a measure of the input signal's power (amplitude) over frequency) EEG spectrogram (spectral power against frequency and time).

### - Drug administration

All the following drugs were purchased from Sigma-Aldrich Ireland (Arklow, Ireland) and delivered by intracerebroventricular (i.c.v.) microinjection in 2 µl volume unless otherwise specified: Adenosine 5'-triphosphate (ATP) at 100 µM, Uridine-5'-triphosphate (UTP) at 100 µM, Uridine-5'-diphosphate (UDP) at 100 µM, 2-chloro-N6-cyclopentyladenosine (CCPA) 300 nM, Pyridoxal-phosphate-6-azophenyl-2,4-disulfonate (PPADS) at 50 µM, α,β- methylene- adenosine-5-triphosphate (α,β-meATP) at 0.1 nmol, 3-O-(4-benzoyl)benzyl-ATP (Bz-ATP) at 300 µM, Brillant Blue G (BBG) at 30 nM.

A-438079 (3-[[5-(2,3-Dichlorophenyl)-1H-tetrazol-1-yl]methyl]pyridine hydrochloride) (50 µM) was from Tocris Biosciences (Bristol, U.K.) and the P2X7R specific antibody was from Alomone Labs (APR-008, Alomone Labs, Ltd, Jerusalem, Israel) and injected at 0.7 mg/ml. Lorazepam (Ativan®, Wyeth, Taplow, U.K.) (6 mg/kg) was administered intraperitoneally (i.p.) at 40 or 60 min after KA.

### - Cell culture

Hippocampal neurons were prepared as described previously (Diaz-Hernandez et al., 2008). Briefly, hippocampi were obtained from E17 mouse embryos and, after dissection and washing three times in Ca²⁺/Mg²⁺-free HBSS, tissue was digested in the same solution with 0.25% trypsin for 15 min at 37°C. Hippocampi were then washed three times in Ca²⁺/Mg²⁺-free HBSS and dissociated with a firepolished Pasteur pipette. Cells were counted, resuspended in plating medium (MEM, 10% fetal bovine serum, 0.6% glucose) and plated at a density of 5,000 cells/cm² on poly-L-lysine coated coverslips (1 mg/ml). After plating, neurons were cultured for 24-72 h in neuronal culture medium (Neurobasal, B-27, glutamax-I). For biochemical experiments, hippocampal neurons were plated on polylysine-coated (1 mg/ml) 60-mm plates at a density of 200,000 cells/cm², and cultured for 72 h in neuronal culture medium. AraC was added at a final concentration of 5 µM after 2 days in culture.

### - Luciferine-luciferase luminiscence assay

Extracellular ATP concentration was measured as previously described (Diez-Zaera et al., 2011). Neurons were grown in 6-well plates at a density of 200,000 cells/cm². Culture medium (100 µl) was collected under various experimental conditions, centrifuged at 600 x g for 5 min at 4°C, and 50 µl aliquots of supernatant were transferred to wells of a 96-well plate placed on ice. The 96-well plate was set in a FLUOstar OPTIMA Microplate Luminometer (BMG LABTECH GmbH, Offenburg, Germany) and 50 µL of rLuciferase/Luciferin (rL/L) Reagent (Promega, Madison, WI) reagent was automatically injected into each well at room temperature (25°C). For luminiscence detection, each well was scanned at 1 Hz for 15 s. To measure extracellular ATP concentration, cultured cells were first bathed in HBM buffer for 1 h at 37°C. Then, cells were pretreated for 30 min with 100 µM ARL 67156, a competitive inhibitor of ecto-ATPases, or with 500 µM *N*-Ethylmaleimide (NEM) a vesicular fusion inhibitor, and their medium collected to measure basal ATP concentration. Thereafter, cells were stimulated by adding 100 µM glutamate for 2 min. In some cases, ATP release was also measured in the presence of A-438079 (1 µM). ATP concentration was determined by comparison with a calibration curve generated with ATP standards diluted in the same buffer as the samples.

### - Ca²⁺ studies and microfluorimetric analysis

Calcium microfluorimetric analysis in single neurons was carried out according to (Diaz-Hernandez et al., 2001). Cultured hippocampal neurons at a density of 5,000 cells/cm² were washed with HBM buffer (140 mM NaCl, 5 mM KCl, 1.2 mM NaH₂PO₄ 1.2 mM NaHCO₃, 1 mM MgCl₂, 10 mM glucose and 10 mM HEPES, pH 7.4) and loaded with Fura-2AM solution (5 µM) for 30 minutes at 37°C. This period facilitated intracellular hydrolysis of Fura-2AM. Subsequently, coverslips were washed with HBM and placed in a superfusion chamber on a Nikon Eclipse TE-2000 microscope (Nikon, Barcelona, Spain), where the cells were continuously superfused with HBM at a rate of 1.2 ml/min. Neurons were either successively stimulated or co-stimulated for 30 s with BzATP (100 µM) and glutamate (100 µM) in the absence or presence of BBG (1 µM). When the antagonist was used, neurons were superfused for 5 min with BBG before BzATP and/or glutamate application. In some experiments, an Mg²⁺-free HBM solution was used, replacing MgCl₂ by glucose at a concentration that conserved the solution osmolarity. A pulse of 60 mM K⁺ was applied at the end of each experiment to confirm viability of neurons under study.

Neurons were visualized using a Plan Fluor x40 S/0.5-1.3 oil lens. The wavelength of the incoming light (340 or 380 nm) was selected with the aid of an Optoscan monochromator (10 nm bandwidth), from Cairn Research (Faversham, UK); 12-bit images were acquired with an ORCA-ER C 47 42-98 CCD camera (Hamamatsu, Barcelona, Spain) controlled by Metafluor 6.3r6 PC software (Universal Imaging Corp., Cambridge, UK). The exposure time was 250 ms for each wavelength and the changing time was less than 5 ms. The images were acquired continuously and buffered in a fast SCSI disk. The time course data represent the average light intensity in a small elliptical region within each cell. The background was subtracted at each wavelength and the 340 / 380 ratio was calculated. The data are represented as the normalized F340/F380 fluorescence ratio, which increases as [Ca²⁺]ᵢ increases.

### - Western blot analysis

Western blotting was performed as previously described (Engel et al., 2010). Whole hippocampus was homogenized in lysis buffer containing a protease inhibitor cocktail (Sigma-Aldrich). Protein concentration was determined and then 20 or 50 µg samples boiled in gel-loading buffer and separated by 10, 12 or 15% sodium dodecyl sulphate-polyacrylamide gel electrophoresis. Proteins were transferred to polyvinylidene difluoride or nitrocellulose membranes. The following primary antibodies were used: β-Actin (Sigma-Aldrich); α-Tubulin (Santa Cruz Biotechnology, Santa Cruz, CA, USA); P2X1R, P2X2R, P2X3R, P2X4R, P2X5R and P2X7R (APR-008 and APR-004) (Alomone Labs) and Interleukin-1β (R&D Systems, Minneapolis, MN, USA). Membranes were next incubated with horseradish peroxidase-conjugated secondary antibodies (Isis Ltd, Bray, Ireland) and protein bands visualized using Supersignal West Pico Chemiluminescent Substrate (Pierce, Rockford, IL, USA). Gel band image densities were captured using a Fuji-film LAS-3000 and analyzed using Alpha-EaseFC4.0 software as described (Engel et al., 2010).

### - Histopathology

Flash-frozen brains in 2-methylbutane were sectioned at -20°C on a Leica cryostat and 12 µm sections collected at the level of dorsal hippocampus (-1.8 mm and - 2.9 mm from Bregma according to a mouse stereotaxic atlas (Paxinos and Franklin, 2001). Neuronal death was assessed using Fluoro-Jade B (FJB) staining, as previously described (Engel et al., 2010; Murphy et al., 2010). Briefly, sections were air-dried and post-fixed in formalin followed by hydrating through graded alcohols. Sections were then rinsed in distilled water and transferred to 0.006% potassium permanganate solution for 15 min. Sections were rinsed again and transferred to a 0.001% FJB solution according to manufacturer's recommendations (Chemicon Europe Ltd, Chandlers Ford, UK). After staining, sections were mounted in DPX (Sigma-Aldrich). For NeuN immunohistochemistry, sections were fixed and permeabilized, blocked in goat serum and incubated overnight with anti-NeuN antibodies (Millipore Ireland B.V., Tullagreen, Ireland) followed by incubation with goat anti-rabbit IgG antibodies coupled to AlexaFluor 568 (BioSciences Limited, Dun Laoghaire, Ireland). Sections were examined using a Nikon 2000s epifluorescence microscope under Ex/Em wavelengths of 472/520 nm (green) and 540 to 580/600 to 660 nm (red) and images captured using a Hamamatsu Orca 285 camera. Cell counts were the average of two adjacent sections for the CA3 subfield under 40x lens magnification by an observer blinded to treatment.

### - Confocal microscopy

For confocal microscopy, animals were transcardially perfused with 4% paraformaldehyde (PFA) in Sorensen's buffer for 10 min. Brains were post-fixed in 4% PFA for 2 h at 4°C and cryoprotected in 30% sucrose solution. 30 µm sagittal or coronal sections were cut on a Leica cryostat and collected in 0.1% azide-PBS solution. Sections were rinsed in PBS-0.1% Triton-X100 and incubated in 1% glycine-PBS-Triton X-100 buffer. Then, slices were treated with PBS containing 0.1% Triton X-100 and 1% fetal calf serum for 30 min. After a brief wash with PBS, slices were incubated for 1 h or overnight with the primary antibodies; anti-NeuN antibody (Milipore), rabbit polyclonal anti-Iba-1 antibody (Wako Chemicals USA, Richmond, VA, USA), mouse monoclonal anti-GFAP antibody (Santa Cruz Biotechnology), anti-P2X7R antibody (Alomone Labs) and mouse monoclonal anti-synaptophysin antibody (Santa Cruz Biotechnology). They were then washed and incubated with the appropriate secondary antibody coupled to AlexaFluor 568 or AlexaFluor 488 (BioSciences Limited, Dun Laoghaire, Ireland). Some sections were also treated with 4^{/}, 6 diamidino-2-phenylindole (DAPI - Vector Laboratories, Burlingame, CA) at the end of the protocol and sections were immediately coverslipped with Fluorosave. Confocal images were acquired with a Leica TCR 6500 microscope equipped with four laser lines (405, 488, 561 and 653 nm) using a 63X immersion oil objective (NA 1.3).

### - Data analysis

Data are presented as means ± SE. Data were analyzed using ANOVA with *post hoc* Fisher's PLSD test or, for 2-group comparison, Student's *t* test (StatView software; SAS Institute, Cary, NC, USA). Significance was accepted at *P <* 0.05.

### - Example 1: Role of ATP-gated receptors in the generation of seizures during SE. Reduction of seizure time by the administration of P2X7R antagonists

Both excitatory and inhibitory effects have been attributed to P2X-receptors in the brain. The authors began by establishing the role of ATP-gated receptors in the generation of seizures during *status epilepticus.*

### 1.1. Glutamate induces vesicular release of ATP

The authors first sought to establish whether glutamate can induce ATP release from hippocampal neurons. In primary cultures, application of glutamate induced the rapid release of ATP from hippocampal neurons *in vitro* (Fig. 2 A, B). ATP release was blocked when neurons were pre-treated with the vesicle fusion release inhibitor, N-Ethylmaleimide (NEM) (Rodriguez et al., 1994) (Fig. 2 A, B). Thus, glutamate-evoked ATP release occurs by a vesicle-dependent manner in hippocampal primary neurons.

### 1.2. ATP potentiates seizures during SE through P2X7R activation

Next, a model of focal-onset SE was used to evaluate the contributions of ATP-gated receptors *in vivo.* Mice received intra-amygdala microinjection of KA to induce SE (Fig. 2C). Seizures began within 5-10 min and clinical features include stereotyped masticatory movements, tonic components (Straub tail: tail being held rigidly and in arched position), forelimb clonus, and episodes of rearing and falling (data not shown). The electroencephalogram (EEG) was recorded by using cranial electrodes (lower part of Fig. 2C). Seizures were curtailed after 40 min by the injection of lorazepam to minimize morbidity and mortality. Typical damage to the hippocampus 24 h later is shown in Fig. 2D, and comprises ipsilateral hippocampal CA3 subfield and hilus neuronal death. No neuronal death is found in the contralateral hippocampus or in the ipsilateral hippocampus of vehicle-injected animals (lower fotograph of Fig. 2D and data not shown).

To explore ATP/purinergic contributions to seizures *in vivo,* mice were co-injected with ATP into the lateral ventricle immediately prior to KA injection (in the area labelled as "Drug" in Fig 2C). This significantly increased the duration of KA-induced high amplitude high frequency discharges (HAHFDs) (Fig. 2E, 2F). Since ATP is rapidly hydrolyzed by ectonucleotidases into different break-down products, finally generating the anticonvulsant adenosine (Burnstock, 2007), it was explored how adenosine receptor activation affected seizure time. Thus, an additional group of animals were co-injected with 2-chloro-N6-cyclopentyladenosine (CCPA), a stable form of adenosine and subject to SE. As expected, in contrast to what was observed when ATP was injected, seizure time was significantly reduced in mice co-injected with CCPA (Fig. 2E, 2F), indicating the pro-convulsive ATP effect is mediated through purinergic P2 receptors.

To elucidate the subclass of P2 receptor mediating the seizure-potentiating effect induced by ATP, the effects of putatively selective P2X and P2Y ligands were examined. Thus, when UTP and UDP, specific P2Y receptor activators (Burnstock, 2007), or the P2X wide-spectrum agonist α/β-methylATP (α/βATP), were co-injected into the ventricle immediately prior to KA injection, no differences in seizure time during SE were observed when compared to vehicle-injected animals (Fig. 2E, 2F, 3A, 3C). However, when mice were co-injected immediately prior to KA with the P2X7R-selective agonist benzoylATP (BzATP) (Burnstock, 2007), a significant increase of seizure time was observed, in a similar manner as was seen for ATP alone (Fig. 3A, 3C).

Next, selective antagonists of P2X receptors were co-injected immediately prior to SE induction. Injection of Brilliant Blue G (BBG) and A-438079, both putatively selective P2X7R antagonists (Burnstock, 2007) significantly reduced seizure time during SE (Fig. 3B,C). In contrast, the general P2X inhibitor pyridoxal-phosphate-6-azophenyl-2,4-disulfonatenon (PPADS) had only a weak, non-significant effect on SE duration (Fig. 3B, C).

To support the P2X7R antagonist findings, an additional group of animals were co-injected with antibodies raised against an extracellular epitope of the P2X7R, prior to KA injection. Specificity of the P2X7R antibody was confirmed in *in vitro* experiments, where the antibody was able to inhibit the P2X7R-gated calcium entry into cultured hippocampal neurons. Compared to control IgG-injected SE mice, animals injected with anti-P2X7R showed significantly reduced seizure time (Fig. 3D).

To explore whether the reduction in seizure time induced by P2X7R antagonists is due to a regulatory action of these compounds on KA receptors, cultures of hippocampal primary neurons were stimulated with BzATP and glutamate in the presence or absence of BBG (Fig. 3E). Analysis of responses to both ligands revealed a high percentage of hippocampal neurons co-express functional P2X7R and glutamate receptors. Moreover, it was also checked that BBG was effective to block the discrete calcium response induced by BzATP, but failed to alter the robust glutamate-induced calcium entry, excluding in this way that glutamate receptors are regulated by P2X7R (Fig. 3E).

### - Example 2: Changes in P2X7R after SE

### 2.1. Selective up-regulation of P2X7R in the hippocampus after SE

To further support the importance of the P2X7R in SE, expression responses of P2X family receptors were compared at different time-points after SE. Hippocampal expression of P2X1,2,3,4,5 and 7 receptors was detected in control mouse brain. Expression levels of P2X2R significantly declined following SE whereas P2X7R levels were significantly increased after SE (Fig. 4 A, B). No changes in protein levels were observed for the remaining P2XR family members analyzed (P2XR1, 3, 4 and 5) after SE (Fig. 4 A, B). P2X6 receptor protein was not detected in mouse hippocampus.

Next, the hippocampus area where P2X7R up-regulation occurred was analyzed. Western blot analysis was performed on additional hippocampal tissue which had been micro-dissected to obtain enriched portions of the CA1, CA3 and DG (Jimenez-Mateos et al., 2008). P2X7R protein levels show a significant increase in the CA1 and DG subfield hippocampus, indicated that it is rapidly up-regulated in the CA1 and DG subfields (Fig. 4C, 4D); P2X7R expression in the CA3 subfield did not significantly change after SE (Fig. 4C, 4D). while no significant changes can be observed in the CA3 subfield

Last, the contralateral hippocampus was examined, a region which is resistant to damage in the model except when seizures are prolonged by delayed treatment with anticonvulsant (Araki et al., 2002). Surprisingly, a significant decrease of P2X7 levels could be observed in the spared, contralateral hippocampus (Fig. 4E, 4F).

### 2.2. P2X7R localization changes after SE

To complement the findings on spatio-temporal changes in P2X7R levels after SE, fluorescence immunohistochemistry was used to localize expression and identify cell types. P2X7 staining was apparent in the CA3 subfield within the dendritic field (stratum radiatum) of the CA3, in the hilus, and within neuronal cell bodies of CA1 (Fig. 5A, 5B). An increase in P2X7R immunoreactivity was observed in animals after SE on the ipsilateral side within the stratum radiatum of the CA1 region and the molecular layer of the dentate gyrus (Fig. 5A, 5B). A decrease in P2X7R immunoreactivity was observed from the cell bodies of CA3 pyramidal neurons and in contralateral subfields, in particular the hilus and CA3 (Fig. 5A, 5B). A re-localization of the P2X7R was also evident, with a transition from a mainly somatic and dendritic localization in control animals (Fig. 5B, 5C) to loss of cell body staining in CA3 along with emergence of P2X7R-stained puncta within dendritic fields of CA3 which were positive for the presynaptic marker synaptophysin (Fig. 5B,C).

Double-fluorescence immunohistochemistry also revealed P2X7R co-localized with NeuN and the microglia marker Iba-1 in control conditions and after SE (Fig. 5B, 5C). Co-localization between the astrocyte marker GF AP and P2X7R was not observed (Fig. 5C).

### - Example 3: Effects of P2X7R antagonism

### 3.1. P2X7R inhibition reduces neuronal death after SE

Having observed that animals injected with P2X7R antagonists displayed reduced seizure time we examined whether hippocampal damage also differed in these mice. Counts of FJB-positive cells within the CA3 subfield of the hippocampus 24 h after SE revealed significantly less damage in mice given BBG prior to SE compared to vehicle-injected SE animals (Fig. 6A, 6B). A second P2X7R antagonist, A-438079, was also found to protect the hippocampus against SE-induced neuronal death (Fig. 6A, 6B). The general P2X receptor antagonist PPADS did not afford protection (Fig. 6A, 6B), indicating that specific blockade of P2X7R reduces hippocampal damage in this SE model.

Neuronal survival between the different treatment groups was compared by assessing numbers of surviving NeuN-stained CA3 cells displaying normal neuronal morphology. Counts of surviving CA3 neurons were significantly higher in mice treated with the P2X7R antagonists BBG and A-438079 when compared to vehicle-injected SE mice (Fig. 6C, 6D). Again, PPADS was not protective (Fig. 6C, 6D).

### 3.2. P2X7R inhibition leads to a diminished microglia response and interleukuin-1β expression

P2X7R is localized on microglia after SE where they regulate activation and release of the inflammatory and pro-convulsive cytokine interleukin-1β (IL-1β) (Rappold et al., 2006; Monif et al., 2009). Therefore, microglia activation and IL-1β production after SE was examined in the model of the present invention, focusing on the CA3 subfield as the main site of pathology in the present model (see Fig. 2D). Western blot analysis demonstrated IL-1β levels rapidly increased after SE within the ipsilateral hippocampus (Fig. 7A, 7B). In contrast, BBG significantly reduced IL-1β induction after SE (Fig. 7B, 7C). Reactive microglia, which featured large somata and thick primary processes that stained for Iba-1, were also significantly reduced after SE in mice given either BBG or A-438079 (Fig. 7D, 7E).

### - Example 4: Early post-treatment with P2X7R antagonists during SE reduces seizure time and hippocampal damage

Next, it was considered important to determine whether P2X7R antagonists can disrupt SE when given after onset of seizures, a more therapeutically-relevant dosing regimen.

Additional mice were injected with the more potent and specific P2X7R antagonist A-438079 i.c.v. 15 min after the injection of KA. This time point corresponds to when all mice are having seizures and have had HAHFDs, and falls within current operational definitions of SE as seizures lasting longer than 5 min (Wasterlain and Chen, 2006). Quantitative analysis of EEG determined that the duration of HAHFDs recorded between after P2X7R antagonist injection until 40 min post-KA when lorazepam was injected was significantly reduced in A-438079-treated mice compared to vehicle-treated SE controls (Fig. 8A). Other electrographic components of the EEG were also changed. Total seizure power during this period was significantly reduced in A-438079-treated mice when compared to vehicle-injected animals (Fig. 8B, 8D, 8E). No changes in the frequency of the EEG were found between groups (Fig. 7C).

A spectral heat map of frequency and amplitude further illustrated that A-438079 decreased seizure amplitude but not EEG band frequency in mice during SE (Fig. 8D, 8E).

To determine whether the seizure-suppressive effects of A-438079 also resulted in neuroprotection, brains from animals were analyzed for FJB and NeuN. Animals injected 15 min after KA with A-438079 showed significantly fewer FJB-positive cells and significantly more NeuN-positive cells in CA3, 24 h after SE (Fig. 8F-I).

### - Example 5: Delayed administration of P2X7R inhibitors given once SE is refractory potentiate the anticonvulsant effects of lorazepam

If first treatment options fail to terminate SE, patients often go into a refractory state of SE and require more aggressive treatments (Lowenstein, 2005).

To model refractory SE, seizures were allowed to continue beyond the normal time when lorazepam is given (40 min) and instead the effects of drugs were recorded 60 min post-KA on the EEG during the next hour.

By 60 min after KA, EEG recordings showed continuous electrographic epileptiform activity which persisted throughout the 60 min recording period (Fig. 9A, 9B, 9E, 9F). Injection of vehicle into these animals had no effect on electrographic seizure activity (Fig. 9A, 9B, 9E, 9F). Injection of A-438079 resulted in some suppression of seizure activity, particularly within the first 15-30 min following injection. HAHfDs continued however, and total seizure time was not significantly different compared to vehicle-only SE mice (Fig. 9A, 9B, 9C). Lorazepam also failed to significantly reduce the duration of HAHFDs when given 60 min after KA, confirming the seizures had become pharmacoresistant. A significant reduction in total seizure power was, however, detected in lorazepam-only treated mice (Fig. 9C, 9F). Thus, a majority of A-438079- and lorazepam-only mice continued in SE (A-438079; 3/5, lorazepam; 4/7).

In contrast, in mice injected at the same time with lorazepam plus A-438079, termination of SE was successful in all mice (5/5) (Fig. 9A-F). EEG recordings confirmed the combination significantly reduced HAHFD duration (Fig. 9A,B) and reduced total EEG power (Fig. 9C, 9E, 9F). No changes in EEG band frequency were observed between treatment groups (Fig. 9D).

Only mice co-injected with lorazepam and A-438079 at the same time showed less seizure-damage in CA3 compared to vehicle- and injection of lorazepam or A-438079 alone.

### - Example 6: P2X7R inhibitors reduce seizure time in a neonatal SE animal model

To explore whether inhibitors of P2X7 receptor might also work against seizures elicited in neonates, the inventors used their recently developed model of focal-onset status epilepticus in 10 day old (P10) rat pups (age equivalent of human neonate) (Dunleavy et al., 2010). Similarly to the mouse model, rat pups were equipped with skull surface EEG and given intra-amygdala microinjection of kainic acid (2 µg) via an implanted guide cannula. P10 rat pups develop SE shortly after KA injection. Seizures in the model are propagated to the hippocampus and surface EEG recordings have previously shown a transition to low amplitude high frequency EEG, developing into high amplitude high frequency epileptiform EEG seizures. The pups display behaviour including initial masticatory movements and salivation, developing to periods of facial and forelimb myoclonus and progressing to the most commonly observed behaviour of wild running, loss of posture and swimming motions. The high amplitude high frequency spiking is sustained and corresponds to wild running and swimming behaviours during recordings 1-2 h after KA. By 4 hours, continuous seizures have abated but the EEG continues to display intermittent lower-amplitude high frequency spiking and high amplitude low frequency spikes even after 24 h. The pathological consequences of neonatal seizures in the model include hippocampal cell death and the animals later display unilateral hippocampal sclerosis.

Rats (n = 10 - 9) were injected intraperitoneally with A-438079 (150 µmol/kg body mass) 40 minutes after the intra-amygdala injection of KA. As rats developed SE shortly after KA injection, it can be considered that they were in a condition of prolonged seizure. Control rats (n= 10 -9) received a intraperitoneal injection of the vehicle, PBS.

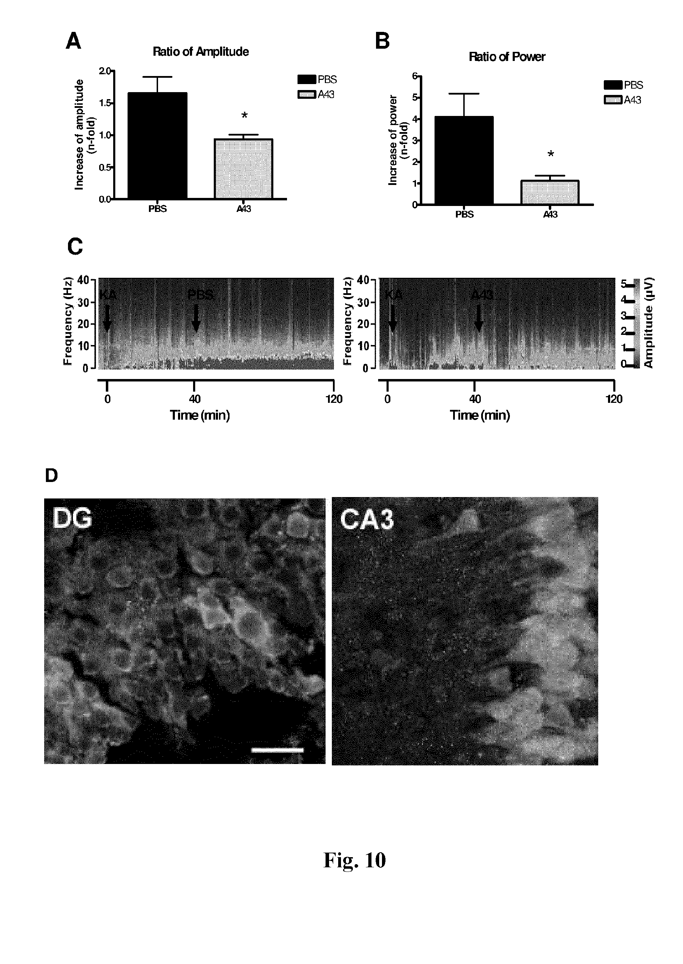

Fig. 10 shows that administration of A-438079 (150 µmol/kg body mass), after a delay of 40 min post-KA, strongly reduced seizures in P10 rat pups. In the control rats, to the contrary, the frequency of seizures even increased after PBS injection (see panel C of Fig. 10). Rats injected with A-438079 showed less amplitude and less total power when compared to vehicle injected p10 rats.

Hippocampal P2X7R staining confirmed the presence of P2X7R in p10 rats 24 hours after SE, P2X7R immunoreactiviy being apparent within the main hippocampal subfields (DG and CA3) (see left photograph in Fig. 10D), co-localized with the neuronal marker NeuN (see right photograph in Fig. 10D).

These data establish that P2X7 receptor antagonists can significantly reduce seizures in an animal model of prolonged neonatal seizures.

### Conclusions

The above examples provide important new insights into the molecular mechanisms of SE and potential pharmacotherapy. They provide evidence supporting a P2X7R contribution to SE which can be targeted to reduce seizure duration and the pathologic consequences of SE. Moreover, and very importantly from the clinical point of view, the data provided show P2X7R targeting is also effective as adjunctive therapy, enhancing the seizure-suppressive effects of lorazepam when given at a time that seizures are partly pharmacoresistant.

### References

Araki T, Simon RP, Taki W, Lan JQ, Henshall DC (2002) Characterization of neuronal death induced by focally evoked limbic seizures in the C57BL/6 mouse. J Neurosci Res 69:614-621.
Atkinson L, Batten TF, Moores TS, Varoqui H, Erickson JD, Deuchars J (2004) Differential co-localisation of the P2X7 receptor subunit with vesicular glutamate transporters VGLUT1 and VGLUT2 in rat CNS. Neuroscience 123:761-768.
Avignone E, Ulmann L, Levavasseur F, Rassendren F, Audinat E (2008) Status epilepticus induces a particular microglial activation state characterized by enhanced purinergic signaling. J Neurosci 28:9133-9144.
Burnstock G (2007) Physiology and pathophysiology of purinergic neurotransmission. Physiol Rev 87:659-797.
Burnstock G (2008) Purinergic signalling and disorders of the central nervous system. Nat Rev Drug Discov 7:575-590.
Chen JW, Wasterlain CG (2006) Status epilepticus: pathophysiology and management in adults. Lancet Neurol 5:246-256.
Cho JH, Choi IS, Jang IS (2010) P2X7 receptors enhance glutamate release in hippocampal hilar neurons. Neuroreport 21:865-870.
DeGiorgio CM, Tomiyasu U, Gott PS, Treiman DM (1992) Hippocampal pyramidal cell loss in human status epilepticus. Epilepsia 33:23-27.
DeLorenzo RJ, Pellock JM, Towne AR, Boggs JG (1995) Epidemiology of status epilepticus. J Clin Neurophysiol 12:316-325.
Deuchars SA, Atkinson L, Brooke RE, Musa H, Milligan CJ, Batten TF, Buckley NJ, Parson SH, Deuchars J (2001) Neuronal P2X7 receptors are targeted to presynaptic terminals in the central and peripheral nervous systems. J Neurosci 21:7143-7152.
Diaz-Hernandez M, Pintor J, Castro E, Miras-Portugal MT (2001) Independent receptors for diadenosine pentaphosphate and ATP in rat midbrain single synaptic terminals. Eur J Neurosci 14:918-926.
Diaz-Hernandez M, del Puerto A, Diaz-Hernandez JI, Diez-Zaera M, Lucas JJ, Garrido JJ, Miras-Portugal MT (2008) Inhibition of the ATP-gated P2X7 receptor promotes axonal growth and branching in cultured hippocampal neurons. J Cell Sci 121:3717-3728.
Diaz-Hernandez M, Diez-Zaera M, Sanchez-Nogueiro J, Gomez-Villafuertes R, Canals JM, Alberch J, Miras-Portugal MT, Lucas JJ (2009) Altered P2X7-receptor level and function in mouse models of Huntington's disease and therapeutic efficacy of antagonist administration. Faseb J 23:1893-1906.
Diez-Zaera M, Diaz-Hernandez JI, Hernandez-Alvarez E, Zimmermann H, Diaz-Hernandez M, Miras-Portugal MT (2011) Tissue-nonspecific alkaline phosphatase promotes axonal growth of hippocampal neurons. Mol Biol Cell 22:1014-1024.
Dona F, Ulrich H, Persike DS, Conceicao IM, Blini JP, Cavalheiro EA, Fernandes MJ (2009) Alteration of purinergic P2X4 and P2X7 receptor expression in rats with temporal-lobe epilepsy induced by pilocarpine. Epilepsy Res 83:157-167.
Dunleavy M, Shinoda S, Schindler C, Ewart C, Dolan R, Gobbo OL, Kerskens CM, Henshall DC (2010). Experimental neonatal status epilepticus and the development of temporal lobe epilepsy with unilateral hippocampal sclerosis. Am J Pathol 176:330-342.
Edwards FA, Gibb AJ, Colquhoun D (1992) ATP receptor-mediated synaptic currents in the central nervous system. Nature 359:144-147.
Ellerkmann RK, Remy S, Chen J, Sochivko D, Elger CE, Urban BW, Becker A, Beck H (2003) Molecular and functional changes in voltage-dependent Na(+) channels following pilocarpine-induced status epilepticus in rat dentate granule cells. Neuroscience 119:323-333.
Engel T, Murphy BM, Hatazaki S, Jimenez-Mateos EM, Concannon CG, Woods I, Prehn JH, Henshall DC (2010) Reduced hippocampal damage and epileptic seizures after status epilepticus in mice lacking proapoptotic Puma. FASEB J 24:853-861.
Evans RJ, Derkach V, Surprenant A (1992) ATP mediates fast synaptic transmission in mammalian neurons. Nature 357:503-505.
Fedele DE, Li T, Lan JQ, Fredholm BB, Boison D (2006) Adenosine A1 receptors are crucial in keeping an epileptic focus localized. Exp Neurol 200:184-190.
Fellin T, Pozzan T, Carmignoto G (2006) Purinergic receptors mediate two distinct glutamate release pathways in hippocampal astrocytes. J Biol Chem 281:4274-4284.
Ferrari D, Villalba M, Chiozzi P, Falzoni S, Ricciardi-Castagnoli P, Di Virgilio F (1996) Mouse microglial cells express a plasma membrane pore gated by extracellular ATP. J Immunol 156:1531-1539.
Ferrari D, Los M, Bauer MK, Vandenabeele P, Wesselborg S, Schulze-Osthoff K (1999) P2Z purinoreceptor ligation induces activation of caspases with distinct roles in apoptotic and necrotic alterations of cell death. FEBS Lett 447:71-75.
Folbergrova J, Ingvar M, Siesjo BK (1981) Metabolic changes in cerebral cortex, hippocampus, and cerebellum during sustained bicuculline-induced seizures. J Neurochem 37:1228-1238.
Franke H, Krugel U, Illes P (2006) P2 receptors and neuronal injury. Pflugers Arch 452:622-644.
Fujikawa DG (1996) The temporal evolution of neuronal damage from pilocarpine-induced status epilepticus. Brain Res 725:11-22.
Fujikawa DG, Itabashi HH, Wu A, Shinmei SS (2000) Status epilepticus-induced neuronal loss in humans without systemic complications or epilepsy. Epilepsia 41:981-991.
Gomez-Villafuertes R, Gualix J, Miras-Portugal MT (2001) Single GABAergic synaptic terminals from rat midbrain exhibit functional P2X and dinucleotide receptors, able to induce GABA secretion. J Neurochem 77:84-93.
Green SM, Cote CJ (2009) Ketamine and neurotoxicity: clinical perspectives and implications for emergency medicine. Ann Emerg Med 54:181-190.
Gualix J, Gomez-Villafuertes R, Diaz-Hernandez M, Miras-Portugal MT (2003) Presence of functional ATP and dinucleotide receptors in glutamatergic synaptic terminals from rat midbrain. J Neurochem 87:160-171.
Henshall DC, Sinclair J, Simon RP (2000) Spatio-temporal profile of DNA fragmentation and its relationship to patterns of epileptiform activity following focally evoked limbic seizures. Brain Res 858:290-302.
Jiang LH, Mackenzie AB, North RA, Surprenant A (2000) Brilliant blue G selectively blocks ATP-gated rat P2X(7) receptors. Mol Pharmacol 58:82-88.
Jimenez-Mateos EM, Hatazaki S, Johnson MB, Bellver-Estelles C, Mouri G, Bonner C, Prehn JH, Meller R, Simon RP, Henshall DC (2008) Hippocampal transcriptome after status epilepticus in mice rendered seizure damage-tolerant by epileptic preconditioning features suppressed calcium and neuronal excitability pathways. Neurobiol Dis 32:442-453.
Jun DJ, Kim J, Jung SY, Song R, Noh JH, Park YS, Ryu SH, Kim JH, Kong YY, Chung JM, Kim KT (2007) Extracellular ATP mediates necrotic cell swelling in SN4741 dopaminergic neurons through P2X7 receptors. J Biol Chem 282:37350-37358.
Karanjia R, Garcia-Hernandez LM, Miranda-Morales M, Somani N, Espinosa-Luna R, Montano LM, Barajas-Lopez C (2006) Cross-inhibitory interactions between GABAA and P2X channels in myenteric neurones. Eur J Neurosci 23:3259-3268.
Khakh BS, North RA (2006) P2X receptors as cell-surface ATP sensors in health and disease. Nature 442:527-532.
Khakh BS, Gittermann D, Cockayne DA, Jones A (2003) ATP modulation of excitatory synapses onto interneurons. J Neurosci 23:7426-7437.
Kim JE, Ryu HJ, Yeo SI, Kang TC (2010) P2X7 receptor differentially modulates astroglial apoptosis and clasmatodendrosis in the rat brain following status epilepticus. Hippocampus.
Labrakakis C, Tong CK, Weissman T, Torsney C, MacDermott AB (2003) Localization and function of ATP and GABAA receptors expressed by nociceptors and other postnatal sensory neurons in rat. J Physiol 549:131-142.
Labrousse VF, Costes L, Aubert A, Darnaudery M, Ferreira G, Amedee T, Laye S (2009) Impaired interleukin-1beta and c-Fos expression in the hippocampus is associated with a spatial memory deficit in P2X(7) receptor-deficient mice. PLoS One 4:e6006.
Lawrence R, Inder T (2010). Neonatal Status Epilepticus. Sim Pediatr Neurol 17: 163-168.
Lowenstein DH (2005) Treatment options for status epilepticus. Curr Opin Pharmacol 5:334-339.
Lowenstein DW, Shimosaka S, So YT, Simon RP (1991) The relationship between electrographic seizure activity and neuronal injury. Epilepsy Res 10:49-54.
Mazarati AM, Wasterlain CG (1999) N-methyl-D-asparate receptor antagonists abolish the maintenance phase of self-sustaining status epilepticus in rat. Neurosci Lett 265:187-190.
McGaraughty S, Chu KL, Namovic MT, Donnelly-Roberts DL, Harris RR, Zhang XF, Shieh CC, Wismer CT, Zhu CZ, Gauvin DM, Fabiyi AC, Honore P, Gregg RJ, Kort ME, Nelson DW, Carroll WA, Marsh K, Faltynek CR, Jarvis MF (2007) P2X7-related modulation of pathological nociception in rats. Neuroscience 146:1817-1828.
Melani A, Amadio S, Gianfriddo M, Vannucchi MG, Volonte C, Bernardi G, Pedata F, Sancesario G (2006) P2X7 receptor modulation on microglial cells and reduction of brain infarct caused by middle cerebral artery occlusion in rat. J Cereb Blood Flow Metab 26:974-982.
Michel AD, Xing M, Thompson KM, Jones CA, Humphrey PPA (2006). Decavanadate, a P2X receptor antagonist, and its use ot study ligand interactions with P2X7 receptors. Br J Pharmacol 534:19-24.
Miras-Portugal MT, Diaz-Hernandez M, Giraldez L, Hervas C, Gomez-Villafuertes R, Sen RP, Gualix J, Pintor J (2003) P2X7 receptors in rat brain: presence in synaptic terminals and granule cells. Neurochem Res 28:1597-1605.
Monif M, Burnstock G, Williams DA (2010) Microglia: proliferation and activation driven by the P2X7 receptor. Int J Biochem Cell Biol 42:1753-1756.
Monif M, Reid CA, Powell KL, Smart ML, Williams DA (2009) The P2X7 receptor drives microglial activation and proliferation: a trophic role for P2X7R pore. J Neurosci 29:3781-3791.
Murphy B, Dunleavy M, Shinoda S, Schindler C, Meller R, Bellver-Estelles C, Hatazaki S, Dicker P, Yamamoto A, Koegel I, Chu X, Wang W, Xiong Z, Prehn J, Simon R, Henshall D (2007) Bcl-w protects hippocampus during experimental status epilepticus. Am J Pathol 171:1258-1268.
Murphy BM, Engel T, Paucard A, Hatazaki S, Mouri G, Tanaka K, Tuffy LP, Jimenez-Mateos EM, Woods I, Dunleavy M, Bonner HP, Meller R, Simon RP, Strasser A, Prehn JH, Henshall DC (2010) Contrasting patterns of Bim induction and neuroprotection in Bim-deficient mice between hippocampus and neocortex after status epilepticus. Cell Death Differ 17:459-468.
Murrell-Lagnado RD, Qureshi OS (2008) Assembly and trafficking of P2X purinergic receptors (Review). Mol Membr Biol 25:321-331.
Naylor DE, Liu H, Wasterlain CG (2005) Trafficking of GABA(A) receptors, loss of inhibition, and a mechanism for pharmacoresistance in status epilepticus. J Neurosci 25:7724-7733.
Nelson DW, Gregg RJ, Kort ME, et al. (2006) Structure-activity relationship studies on a series of novel, substituted 1-benzyl-5-phenyltetrazole P2X7 antagonists. JMed Chem.;49(12):3659-3666*.*
Nieber K, Eschke D, Brand A (1999) Brain hypoxia: effects of ATP and adenosine. Prog Brain Res 120:287-297.
North RA (2002) Molecular physiology of P2X receptors. Physiol Rev 82:1013-1067 Pankratov Y, Castro E, Miras-Portugal MT, Krishtal O (1998) A purinergic component of the excitatory postsynaptic current mediated by P2X receptors in the CA1 neurons of the rat hippocampus. Eur J Neurosci 10:3898-3902.
Papp L, Vizi ES, Sperlagh B (2004) Lack of ATP-evoked GABA and glutamate release in the hippocampus of P2X7 receptor-/- mice. Neuroreport 15:2387-2391.
Paxinos G, Franklin KBJ (2001) The mouse brain in stereotaxic coordinates, second edition. San Diego, CA: Elsevier.
Pisani F, Cerminara C, Fusco C, Sisti L (2007) Neonatal status epilepticus vs recurrent neonatal seizures - Clinical findings and outcome. Neurology 69: 2177-2185.
Rappold PM, Lynd-Balta E, Joseph SA (2006) P2X7 receptor immunoreactive profile confined to resting and activated microglia in the epileptic brain. Brain Res 1089:171-178.
Ravizza T, Lucas SM, Balosso S, Bernardino L, Ku G, Noe F, Malva J, Randle JC, Allan S, Vezzani A (2006) Inactivation of caspase-1 in rodent brain: a novel anticonvulsive strategy. Epilepsia 47:1160-1168.
Robson SC, Sevigny J, Zimmermann H (2006) The E-NTPDase family of ectonucleotidases: Structure function relationships and pathophysiological significance. Purinergic Signal 2:409-430.
Rodriguez L, Stirling CJ, Woodman PG (1994) Multiple N-ethylmaleimide-sensitive components are required for endosomal vesicle fusion. Mol Biol Cell 5:773-783.
Rona S, Rosenow F, Arnold S, Carreno M, Diehl B, Ebner A, Fritsch B, Hamer HM, Holthausen H, Knake S, Kruse B, Noachtar S, Pieper T, Tuxhorn I, Luders HO (2005) A semiological classification of status epilepticus. Epileptic Disord 7:5-12.
Skaper SD, Debetto P, Giusti P (2010) The P2X7 purinergic receptor: from physiology to neurological disorders. Faseb J 24:337-345.
Sloviter RS, Zappone CA, Bumanglag AV, Norwood BA, Kudrimoti H (2007) On the relevance of prolonged convulsive status epilepticus in animals to the etiology and neurobiology of human temporal lobe epilepsy. Epilepsia 48 Suppl 8:6-10.
Sokolova E, Nistri A, Giniatullin R (2001) Negative cross talk between anionic GABAA and cationic P2X ionotropic receptors of rat dorsal root ganglion neurons. J Neurosci 21:4958-4968.
Sperlagh B, Vizi ES, Wirkner K, Illes P (2006) P2X7 receptors in the nervous system. Prog Neurobiol 78:327-346.
Sun SH (2010) Roles of P2X7 receptor in glial and neuroblastoma cells: the therapeutic potential of P2X7 receptor antagonists. Mol Neurobiol 41:351-355.
Ubogu EE, Sagar SM, Lerner AJ, Maddux BN, Suarez JI, Werz MA (2003) Ketamine for refractory status epilepticus: a case of possible ketamine-induced neurotoxicity. Epilepsy Behav 4:70-75.
Vezzani A, Conti M, De Luigi A, Ravizza T, Moneta D, Marchesi F, De Simoni MG (1999) Interleukin-1beta immunoreactivity and microglia are enhanced in the rat hippocampus by focal kainate application: functional evidence for enhancement of electrographic seizures. J Neurosci 19:5054-5065.
Vianna EP, Ferreira AT, Naffah-Mazzacoratti MG, Sanabria ER, Funke M, Cavalheiro EA, Fernandes MJ (2002) Evidence that ATP participates in the pathophysiology of pilocarpine-induced temporal lobe epilepsy: fluorimetric, immunohistochemical, and Western blot studies. Epilepsia 43 Suppl 5:227-229.
Wang X, Arcuino G, Takano T, Lin J, Peng WG, Wan P, Li P, Xu Q, Liu QS, Goldman SA, Nedergaard M (2004) P2X7 receptor inhibition improves recovery after spinal cord injury. Nat Med 10:821-827.
Wasterlain CG, Chen JW (2006) Definition and classification of status epilepticus. In: Status epilepticus: mechanisms and management (Wasterlain CG, Treiman DM, eds), pp 11-16. Cambridge: MIT Press.
Wasterlain CG, Chen JW (2008) Mechanistic and pharmacologic aspects of status epilepticus and its treatment with new antiepileptic drugs. Epilepsia 49 Suppl 9:63-73.
Wasterlain CG, Liu H, Naylor DE, Thompson KW, Suchomelova L, Niquet J, Mazarati AM, Baldwin RA (2009) Molecular basis of self-sustaining seizures and pharmacoresistance during status epilepticus: The receptor trafficking hypothesis revisited. Epilepsia 50 Suppl 12:16-18.
Wieraszko A, Goldsmith G, Seyfried TN (1989) Stimulation-dependent release of adenosine triphosphate from hippocampal slices. Brain Res 485:244-250.
Yu Y, Ugawa S, Ueda T, Ishida Y, Inoue K, Kyaw Nyunt A, Umemura A, Mase M, Yamada K, Shimada S (2008) Cellular localization of P2X7 receptor mRNA in the rat brain. Brain Res 1194:45-55.
Ziemann AE, Schnizler MK, Albert GW, Severson MA, Howard MA, 3rd, Welsh MJ, Wemmie JA (2008) Seizure termination by acidosis depends on ASIC1a. Nat Neurosci 11:816-822.
Zimmermann H (2000) Extracellular metabolism of ATP and other nucleotides. Naunyn Schmiedebergs Arch Pharmacol 362:299-309.

## Claims

1. An antagonist of P2X7 receptor for use in the treatment of *status epilepticus.*

2. An antagonist of P2X7 receptor for use according to claim 1, wherein the antagonist is a selective antagonist.

3. An antagonist of P2X7 receptor, for use according to claim 1, wherein the
antagonist is a disubstituted tetrazole of the Formula I, 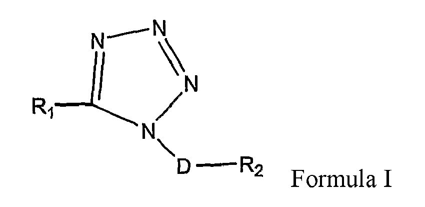 wherein,
R1 is selected of the groups consisting of cycloalkenyl, cycloalkyl, Heterocyclo, aryl, Heteroaryl, arylalkyl and Heteroarylalkyl; wherein each R1 is substituted with 0, 1, 2, 3, 4 or 5 sustituents which are independently selected from the group consisting of alkyl, alkenyl, alkinyl, nitro, cyano, Halo, -OR_{C}, -O(CO)R_{c}, -OC(O)OR_{c}, -OS(O)₂R_{c}, - SR_{C}, -S(O)R_{c} -S(O)₂R_{C}, -S(O)₂OR_{c}, -S(O)₂NR_{c}R_{d} , -NR_{c}R_{d}, -N(R_{d})C(O)OR_{c}, - N(R_{d})C(O)NR_{c}R_{d}, -N (R_{d})S(O)₂NR_{c}P_{d}, -C(O)R_{c}, -C(O)OR_{c}, -C(O)NR_{c}R_{d}, haloalkyl, cianoalkyl, nitroalkyl, -alkylOR_{c}, -O(CO)R_{c}, -alkylOC(O)OR_{c}, -alkylOS(O)₂R_{c}, alkylSR_{c}-, -alkylS(O)R_{c}, -alkylS(O)₂R_{C}, -alkylS(O)₂OR_{c}, -alkylS(O)₂NR_{c}R_{d}, alkylNR_{c}R_{d}, -alkylN(R_{d})C(O)OR_{C}, -alkylN(R_{d})C(O)NR_{c}R_{d}, -alkylN(R_{d})S(O)₂NR_{c}R_{d}, alkylC(O)R_{c}, -alkylC(O)OR_{C}, -alkylC(O)NR_{c}R_{d} and R3; provided that, when R1 is arylalkyl or Heteroarylalkyl, D is a bond;
R2 is selected of the groups consisting of cycloalkyl, cycloalkenyl, Heterocyclo, aryl and Heteroaryl; wherein each R2 is substituted with 0, 1, 2, 3, 4 or 5 sustituents which are independently selected from the group consisting of alkyl, alkenyl, alkinyl, nitro, cyano, Halo, -OR_{C}, -O (CO)R_{c}, -OC(O)OR_{c}, -OS(O)₂R_{C}, -SR_{C}, -S(O)R_{c}, - S(O)₂R_{C}, -S(O)₂OR_{c}, -S(O)₂NR_{c}R_{d} , -NR_{c}R_{d}, -N(R_{d})C(O)OR_{c}, -N(R_{d})C(O)NR_{c}R_{d}, - N(R_{d})S(O)₂NR_{c}R_{d}, -C(O)R_{c}, -C(O)OR_{c}, -C(O)NR_{c}R_{d}, Haloalkyl, cyanoalkyl, nitroalkyl, -alkylR_{c}, -O(CO)R_{c}, -alkylC(O)OR_{C}, -alkylS(O)₂R_{C}, alkylSR_{c}, -alkylS(O)R_{c},-alkylS(O)₂R_{C},-alkylS(O)₂OR_{c},-alkylS(O)₂NR_{c}R_{d}, alkylNR_{c}R_{d}, -alkylN(R_{d})C(O)OR_{C}, - alkyllN(R_{d})C(O)NR_{c}R_{d}, -alkylN(R_{d})S(O)₂NR_{c}R_{d}, alkylC(O)R_{c}, -alkylC(O)OR_{C}, - alkylC(O)NR_{c}R_{d} and R3;
R3 si selected of the group consisting of cycloalkyl, cycloalkenyl, Heterocyclo, aryl and Heteroaryl; wherein each R3 is substituted with 0, 1, 2, 3, 4 or 5 substituents which are independently selected from the group consisting of alkyl, alkenyl, alkinyl, nitro, cyano, Halo, formyl, Hydroxy, alkoxy, Haloalkoxy, -OC(O)alkyl, -S(O)₂alkyl, - S(O)₂NH₂, -S(O)₂N(H)(alkyl), -S(O)₂N(alkyl)₂, -NH₂, -N(H)(alkyl), -N(alkyl)₂, - C(O)(alkyl), -C(O)(OH), -C(O)(Oalkyl), -C(O)NH₂, -C(O)N(H)(alkyl), -C(O)N(alkyl)₂, Haloalkyl, formylalkyl, cyanoalkyl, nitroalkyl, Hydroxyalkyl, alkoxyalkyl, Haloalkoxyalkyl, -alkylC(O)alkyl, -alkyl-S(O)₂alkyl, -alkyl-S(O)₂NH₂, -alkylS(O)₂N(H)(alkyl), -alkyl-S(O)₂N(alkyl)₂, -alkyl-NH₂, -alkyl-N(H)(alkyl), -alkyl-N(alkyl)₂, -alkyl-C(O)(alkyl),- alkyl-C(O)(OH), -alkyl-C(O)(Oalkyl), -alkyl-C(O)NH₂, - alkyl-C(O)N(H)(alkyl), and -alkyl-C(O)N(alkyl)₂; and
R_{c} y R_{d}, in each occurrence, are independently selected from the group consisting of hydrogen, alkyl, haloalkyl, aryl, heteroaryl, arylalkyl and heteroarylalkyl; wherein the aryl, heteroaryl, aryl of the arylalkyl moiety and heteroaryl of the heteroarylalkyl moieties are independently substituted with 0, 1, 2 or 3 substituents which are independently selected from the group consisting of alkyl, halo, haloalkyl, hydroxy, hydroxyalkyl and alkoxialkyl.

4. An antagonist of P2X7 receptor, for use according to claim 3, wherein the antagonist is a disubstituted tetrazole selected from one of the following groups:
a) the disubstituted tetrazoles of Formula II 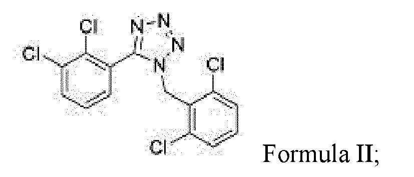
b) the substituted tetrazoles of Formula III 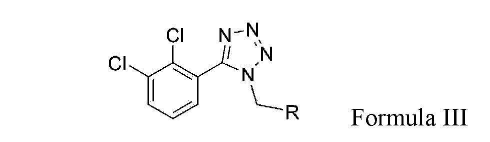 wherein R is selected from the group of C₆H₅, 2-CH₃-C₆H₆, 2-pyridyl, 3-pyridiyl, 4-pyridyl, 2-CH₃-3-pyridyl, 3-pyridinylmethyl, 2,4-(CH₃)₂-5-thiazolyl, 3,5-(CH₃)₂-4-isoxazolyl;
c2,3-(CH₃)₂-C₆H₃, 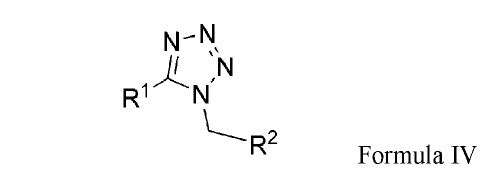 wherein,
R² and R¹ are selected so that
when R² is 3-pyridyl, R¹ is selected from the group of C₆H₅, 2-Cl-C₆H₄, 3-Cl-C₆H₄, 2,5-Cl₂-C₆H₃, 3,4-Cl₂-C₆H₃, 2-CF₃-3-F-C₆H₃, 2-Cl-3-CF₃-C₆H₃, 2-F-3-CF₃-C₆H₃, 2,3-Cl₂-4-F-C₆H₂, 2,3,4-Cl₃- C₆H₂,
when R² is C₆H₅, R¹ is selected from the group of 4-pyridyl, 5-quinolyl, 8-quinolyl, 2,3-Cl₂-4-pyrrolidinyl-C₆H₂,
when R² is 2-CH₃-C₆H, R¹ is 2,3-(CH₃)₂-C₆H₃,
when R² 2-Cl-C₆H₄, R¹ is 2,3-(OCH₃)₂-C₆H₃;
d) the substituted tetrazoles of Formula V, 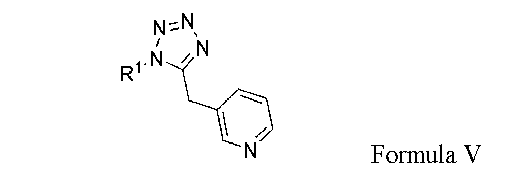 wherein R¹ is selected from the group of 2,3-Cl₂-C₆H₃, 2,3-Cl₂-4-F-C₆H₂, 2-Cl-3-CF₃-C₆H₃, 2-Cl-3-CF₃-4-F-C₆H₂;
e) 1-(2,3-dichlorophenyl)-N-[2-(pyridin-2-yloxy)benzyl]-1H-tetrazol-5-amine (A-839977);
or any pharmaceutically acceptable salt thereof.

5. An antagonist of P2X7 receptor for use according to claim 1 or 2, wherein the antagonist is capable to cross the blood brain barrier.

6. An antagonist of P2X7 receptor for use according to claim 5, wherein the antagonist is selected of the group of Brilliant Blue G (BBG), KN-62, A-438079 (3-[[5-(2,3-dichlorophenyl)-1*H*-tetrazole-1-yl]methyl]pyridine), or any pharmaceutically acceptable salt of decavanadate ((V₁₀O₂₈)⁶⁻).

7. An antagonist of P2X7 receptor for use according to any one of claims 1 to 6, wherein the subject suffering from *status epilepticus* is an adult, a child, an infant or a neonate.

8. An antagonist of P2X7 receptor for use according to any one of claims 1 to 7, wherein the antagonist of the P2X7 receptor is administered in adjunction with an anti-convulsant or any other anti-epileptic drug.

9. An antagonist of P2X7 receptor for use according to claim 8, wherein the antagonist of the P2X7 receptor is administered in adjunction with a benzodiapezine.

10. An antagonist of P2X7 receptor for use according to claim 9, wherein the antagonist of the P2X7 receptor is administered in adjunction with lorazepam.

11. An antagonist of P2X7 receptor for use according to any one of claims 8 to 10, wherein the antagonist is administered prior to, contemporaneous with or subsequent to the administration of the anti-convulsant.

12. An antagonist of the P2X7 receptor for use according to any of the preceding claims, wherein the *status epilepticus* is refractory *status epilepticus.*

13. An antagonist of the P2X7 receptor for use according to claim 12, wherein the antagonist is A-438079, which is administered in adjunctive treatment with lorazepam.

14. A method of use of an antagonist for treating or preventing the seizure in the *status epilepticus* in an animal, which method comprises administering to said animal an effective amount of the antagonist of P2X7 receptor.

15. The method according to claim 14, wherein the antagonist is administered to the animal prior to, contemporaneous with, or subsequent to start of the seizure.

16. The method according to claim 14 or 15, wherein the antagonist of P2X7R is administered in combination with an anti-epileptic drug..

17. The method according to claim 16, wherein the antagonist of P2X7R is administered prior to, simultaneously to, or subsequent to the administration of the anti-epileptic drug.

18. The method according to claim 16 or 17, wherein the anti-epileptic drug is a benzodiazepine.

19. The method according to claim 18, wherein the anti-epileptic drug is lorazepam.

20. The method according to any one of the claims 14 to 19, wherein the antagonist of P2X7R is administered intraperitoneally or intravenously

21. The method of claim 20, wherein the antagonist of P2X7 is capable to cross the blood brain barrier.

22. The method according to any of the claims 14 to 21, for treating or preventing a seizure in refractory *status epilepticus.*

23. The method according to claim 22, wherein the antagonist is administered in combination with a benzodiazepine.

24. The method according to any one of claims 14 to 23, wherein the animal to treat is a human being.

25. The method according to claim 24, wherein the human being is an adult, a children, an infant or a neonate.

26. A method of use of an antagonist of P2X7R for treating or preventing neonatal seizures or seizures in infants or in children up to five years of age, wherein the antagonist of P2X7R is administered alone or in combination with another approved treatment for neonatal seizures such as a barbiturate, a benzodiazepine, any other approved anti-epileptic drug, or in-trial medication such as bumetanide.
